# EUROPEAN PATENT APPLICATION

(11) **EP 3 747 985 A1**
(43) Date of publication of application: **09.12.2020**
(21) Application number: 19747524.7
(22) Date of filing: 24.01.2019
(51) Int. Cl.: C12M 3/00, C12Q 1/02

(54) **CELL CULTURE CONTAINER**

(30) Priority: 01.02.2018 JP 2018016737
(71) Applicant: AGC INC., Chiyoda-ku, Tokyo 1008405 (JP)
(72) Inventor: KOGUCHI Ryohei, Tokyo 100-8405 (JP); EGUCHI Hajime, Tokyo 100-8405 (JP); ZHU Lijun, Tokyo 100-8405 (JP); YAMAMOTO Kyoko, Tokyo 100-8405 (JP)
(74) Representative: Müller-Boré & Partner Patentanwälte PartG mbB
(86) International application number: PCT/JP2019/002323
(87) International publication number: WO 2019/151114

(57) **Abstract**

To provide a cell culture container capable of preparing spheroids having a uniform size highly efficiently, and capable of microscopic observation simply, particularly fluorescence microscopic observation.

A cell culture container comprising:
side walls forming an opening,
a bottom comprising a light-transmitting glass material, closing the lower end of the opening and having a plurality of recesses in a region which the opening faces on its upper surface, and
a surface layer inhibiting cell adhesion formed on the inner surface of the recesses.

## Description

### TECHNICAL FIELD

The present invention relates to a cell culture container. Particularly, it relates to a cell culture container capable of preparing spheroids with uniform sizes highly efficiently and capable of microscopic observation simply.

### BACKGROUND ART

New culture methods to acquire cells having functions close to those in vivo by imitating the form and the environment surrounding cells in vivo have been developed. Particularly, spheroid (cell aggregate) culture technique is an excellent method which can maintain cell interactions, and its application is expected to drug discovery screening of preparing cardiomyocyte or cancer cell spheroids and examining medicinal effects and toxicity. In recent years, in addition to a technique to form spheroids, a technique to control the size of the spheroids has attracted attention.

For example, Patent Document 1 discloses a technique to prepare spheroids in a large amount controlling their sizes by using a cell culture container having a plurality of recesses capable of accommodating cells and of cultivating and observing the cells, with the size and the shape of the recesses defined. In Patent Document 1, a resin made cell culture container is used to define the size and the shape of the recesses, however, in drug discovery screening, if a resin made cell culture container is used, the fluorescence intensity due to the container material is high, and high precision observation is difficult. A low fluorescent cell culture container capable of preparing spheroids and capable of observation with high precision or capable of observing intercellular fluorescence immediately with high precision, has been required.

### PRIOR ART DOCUMENTS

### PATENT DOCUMENT

Patent Document 1: WO2014/196204

### DISCLOSURE OF INVENTION

### TECHNICAL PROBLEM

Under these circumstances, the object of the present invention is to provide a cell culture container capable of preparing spheroids with uniform sizes highly efficiently and capable of microscopic observation simply, particularly fluorescence microscopic observation.

### SOLUTION TO PROBLEM

The present invention provides the following constitutions.
[1] A cell culture container comprising:
   side walls forming an opening,
   a bottom comprising a light-transmitting glass material, closing the lower end of the opening and having a plurality of recesses in a region which the opening faces on its upper surface, and
   a surface layer inhibiting cell adhesion formed on the inner surface of the recesses.
[2] The cell culture container according to [1], wherein the proportion of the total area of the recesses in a plan view to the whole area of the region which the opening faces on the upper surface of the bottom in a plan view, is at least 40%.
[3] The cell culture container according to [1] or [2], which has a plurality of structures each having the opening and the plurality of recesses having the surface layer formed thereon, in a region which the opening faces on the upper surface of the bottom.
[4] The cell culture container according to any one of [1] to [3], wherein a value obtained by dividing the fluorescence intensity at 584 nm when the glass material is excited by light at 532 nm by the fluorescence intensity at 584 nm when quartz glass is excited by light at 532 nm, measured by a 100 magnification objective lens by means of microscopic Raman spectroscopy, is at most 10.
[5] The cell culture container according to any one of [1] to [4], wherein the surface layer has a covalent bond with the bottom.
[6] The cell culture container according to any one of [1] to [5], wherein the amount of the total organic carbon (TOC) eluted from the surface layer into water per unit area 1 cm² of the surface layer, when the surface layer is dipped in water at 40°C for 7 days, is at most 10 mg/L.
[7] The cell culture container according to any one of [1] to [6], which has a silicon oxide layer between the surface layer and the bottom.
[8] The cell culture container according to any one of [1] to [7], wherein the surface layer has a biocompatible group.
[9] The cell culture container according to [8], wherein the biocompatible group comprises at least one type selected from the group consisting of a group represented by the following formula 1, a group represented by the following formula 2 and a group represented by the following formula 3.
[10] A drug discovery screening method, using the cell culture container as defined in any one of [1] to [9].

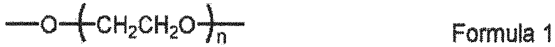

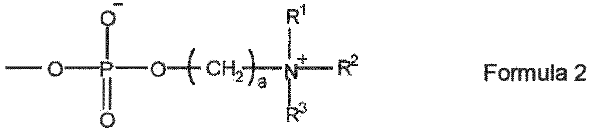

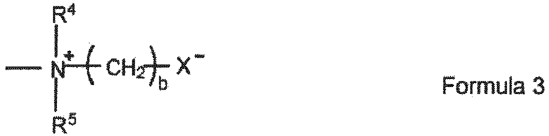

In the formula 1, n is an integer of from 1 to 300, and from 50 to 100 mol% of the group represented by the formula 1 is the group represented by the formula 1 in a group represented by the following formula 4, n in the formula 4 is an integer of from 1 to 300, and R⁶ is a hydrogen atom or a C₁₋₅ alkyl group.

In the formula 2, R¹ to R³ are each independently a C₁₋₅ alkyl group, and a is an integer of from 1 to 5.

In the formula 3, R⁴ and R⁵ are each independently a C₁₋₅ alkyl group, X⁻ is a group represented by the following formula 3-1 or a group represented by the following formula 3-2, and b is an integer of from 1 to 5.

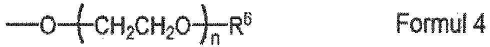

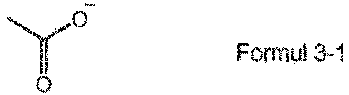

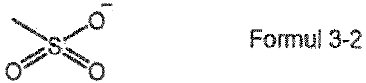

### ADVANTAGEOUS EFFECTS OF INVENTION

According to the present invention, it is possible to provide a cell culture container capable of preparing spheroids with uniform sizes highly efficiently and capable of microscopic observation simply, particularly fluorescence microscopic observation.

### BRIEF DESCRIPTION OF DRAWINGS

Fig. 1 is a plan view illustrating an example of a cell culture container according to an embodiment of the present invention.
Fig. 2 is a schematic cross sectional view illustrating the cell culture container shown in Fig. 1 at the line X-X.
Fig. 3A is a plan view illustrating the bottom of the cell culture container shown in Fig. 1.
Fig. 3B is a cross sectional view illustrating the bottom shown in Fig. 3A at the line X-X.
Fig. 4 is a plan view illustrating another example of a cell culture container according to an embodiment of the present invention.
Fig. 5 is a graph illustrating the distribution of the diameters of spheroids obtained in the cell culture container in Examples.

### DESCRIPTION OF EMBODIMENTS

Now, the present invention will be described with reference to drawings. It should be understood that the present invention is by no means restricted thereto. Other embodiments are also included in the present invention so long as they are within the scope of the present invention. Further, embodiments comprising an optional combination of the following embodiments and modified examples are also preferred examples.

In this specification, a compound and a group represented by a formula are represented also as a compound and a group with the number of the formula, for example, a compound represented by formula 1 is represented also as compound 1.

In this specification, " to " used to show the range of the numerical values is used to include the lower and upper limit values.

A "(meth)acrylate" generally means an acrylate and a methacrylate.

"Units" in a copolymer mean a moiety derived from a monomer formed by polymerization of the monomer.

A "biocompatible group" means a group having a property to inhibit cells from being bonded to a material surface and becoming immobile.

A "cell" is the most fundamental unit constituting a living body and means one which has, in the interior of the cell membrane, the cytoplasm and various organelles. Nuclei containing DNA may be contained or may not be contained inside the cell.

Animal-derived cells include germ cells (sperm, ova, etc.), somatic cells constituting a living body, stem cells, progenitor cells, cancer cells separated from a living body, cells (cell line) which are separated from a living body and have won immortalized ability and thus are stably maintained outside the body, cells separated from a living body and artificially genetically engineered, cells separated from a living body and having nuclei artificially replaced, etc.

Somatic cells constituting a living body include fibroblasts, bone marrow cells, B lymphocytes, T lymphocytes, neutrophils, erythrocytes, platelets, macrophages, monocytes, bone cells, bone marrow cells, pericytes, dendritic cells, keratinocytes, fat cells, mesenchymal cells, epithelial cells, epidermal cells, endothelial cells, vascular endothelial cells, hepatocytes, cartilage cells, cumulus cells, neural cells, glial cells, neurons, oligodendrocytes, microglia, astrocytes, cardiac cells, esophagus cells, muscle cells (for example, smooth muscle cells, skeletal muscle cells), pancreatic beta cells, melanin cells, hematopoietic progenitor cells, mononuclear cells, etc.

The somatic cells include cells taken from optional tissues, such as skin, kidneys, spleen, adrenal gland, liver, lung, ovary, pancreas, uterus, stomach, colon, small intestine, large intestine, bladder, prostate, testis, thymus, muscle, connective tissue, bone, cartilage, vascular tissue, blood, heart, eye, brain, nervous tissue, etc.

The stem cells are cells having both an ability to replicate themselves and an ability to be differentiated into cells of other multiple systems, and include embryonic stem cells (ES cells), embryonic carcinoma cells, embryonic germ stem cells, induced pluripotent stem cells (iPS cells), neural stem cells, hematopoietic stem cells, mesenchymal stem cells, liver stem cells, pancreatic stem cells, muscle stem cells, germ stem cells, intestinal stem cells, cancer stem cells, hair follicle stem cells, etc.

The progenitor cells are cells at an intermediate stage during differentiation into specific somatic or germ cells from the stem cells.

The cancer cells are cells that have acquired an unlimited proliferative capacity as derived from somatic cells.

A cell line is cells which have acquired an unlimited proliferative capacity by an artificial manipulation in vitro, and includes HCT116, Huh7, HEK293 (human embryonic kidney cells), HeLa (human cervical carcinoma cell line), HepG2 (human liver cancer cell line), UT7/TPO (human leukemia cell line), CHO (Chinese hamster ovary cell line), MDCK, MDBK, BHK, C-33A, HT-29, AE-1, 3D9, Ns0/1, Jurkat, NIH3T3, PC12, S2, Sf9, Sf21, High Five, Vero, etc.

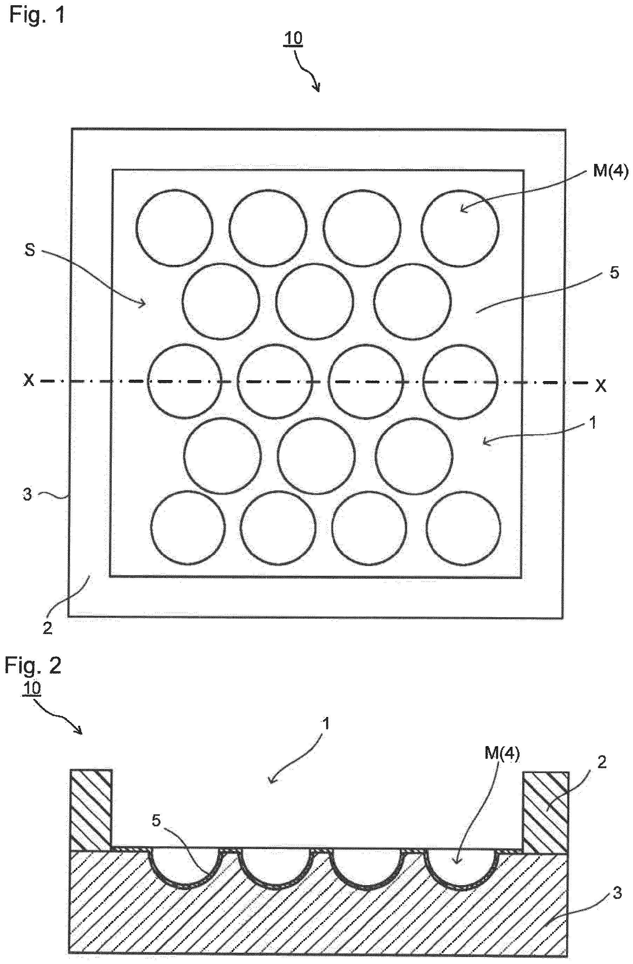

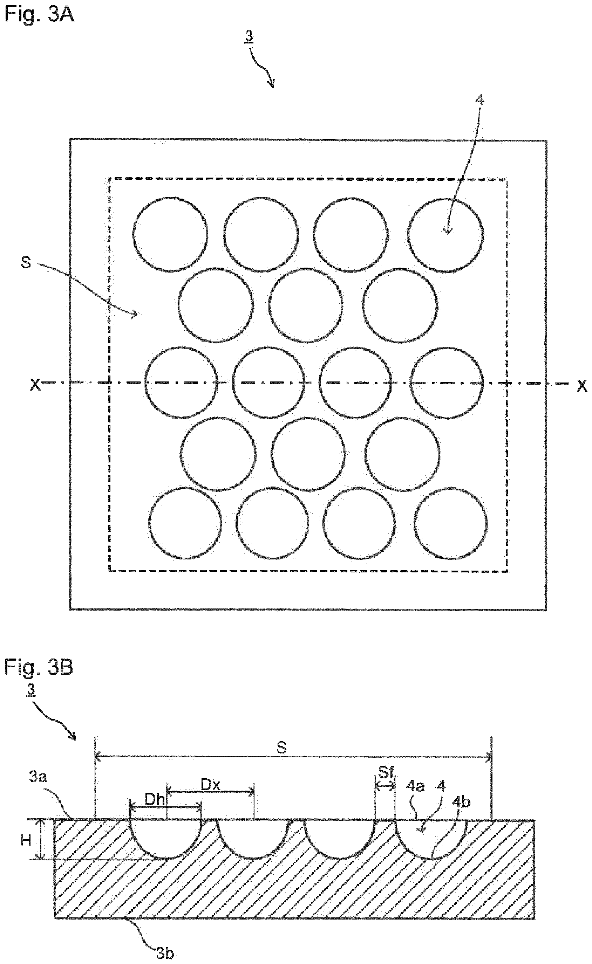

Fig. 1 is a plan view schematically illustrating an example of a cell culture container according to an embodiment of the present invention, and Fig. 2 is a schematic cross sectional view illustrating the cell culture container shown in Fig. 1. Fig. 3A is a plan view illustrating the bottom, and Fig. 3B is a cross sectional view illustrating the bottom shown in Fig. 3A at the line X-X. The cell culture container of the present invention is used, specifically, for preparation of spheroids, by culturing cells to be cultured and in the process of culturing three-dimensionally aggregating the cells to obtain spheroids having desired sizes.

The cell culture container 10 shown in Figs. 1 and 2 comprises side walls 2 forming an opening 1, and a bottom 3 closing the lower end of the opening 1, comprising a light-transmitting glass material. As shown in Figs. 3A and 3B, the bottom 3 has a plurality of recesses 4 in a region S which the opening 1 faces on an upper surface 3a of the bottom 3. In the cell culture container 10, the upper surface 3a of the bottom 3 has a structure such that the whole surface of the region S which the opening 1 faces, including the inner surface of the recesses 4, is covered with a surface layer 5 inhibiting the cell adhesion.

In the bottom 3, the upper surface 3a excluding the recesses 4 and the whole lower surface 3b facing the upper surface 3a are flat. In the bottom 3, the lower surface 3b and the upper surface 3a excluding the recesses 4 are in parallel with each other, and the entire bottom 3 is substantially in a plate shape. Hereinafter, a region excluding the recesses 4 in the region S of the upper surface 3a will be referred to as a flat region Sf. The bottom 3 may be in a plate shape of which the lower surface 3b and the flat region Sf have the same curvature, as the case requires. However, in a case where the cell culture container 10 is used for microscopic observation such as fluorescence microscopic observation as it is after the cell culture, the entire bottom 3 is preferably substantially in a plate shape.

The thickness of the surface layer 5 is basically very small as compared with the thickness of the bottom 3, and the surface shape of the surface layer 5 formed on the upper surface 3a of the bottom 3 in the region S follows the surface shape of the upper surface 3a of the bottom 3. Accordingly, the shape of the upper surface 3a of the bottom 3 can be replaced with the surface shape of the surface layer 4 as it is. In the cell culture container 10, cells are cultured mainly in microspaces M each surrounded by the recessed surface of the surface layer 5 formed on the inner surface of each recess 4 and a plane extending on the recess 4 from the surface of the surface layer 5 formed on the flat region Sf on the upper surface 3a of the bottom 3. Each microspace M can be regarded as having the same shape and size as those of a space which each recess 4 of the bottom have in their inside (a space surrounded by the inner surface of the recess 4 and a recess opening surface 4a). Accordingly, in Figs. 1 and 2, the microspace is represented as M(4) which is a combination of the reference symbol M representing the microspace itself and the reference symbol 4 representing the recess 4. Here, the recess opening surface 4a is an opening surface constituting the upper edge of the recess 4.

The thickness of the bottom 3 is a distance between the flat region Sf of the upper surface 3a and the lower surface 3b and is preferably at least 0.3 mm and at most 1.75 mm in view of easy microscopic observation and sufficient strength. The thickness of the bottom 3 is more preferably at least 0.35 mm, further preferably at least 0.45 mm. The thickness of the bottom 3 is more preferably at most 1.70 mm, further preferably at most 1.50 mm. In view of easy microscopic observation and the strength of the cell culture container, the thickness of the bottom 3 is more preferably at least 0.35 mm and at most 0.70 mm, further preferably at least 0.40 mm and at most 1.50 mm.

The shape and the size of the recesses 4 are properly adjusted depending upon the type of the cells to be cultured, the size of the desired spheroids, the culture conditions, etc. With a view to efficiently preparing spheroids having the desired size, the shape of each recess 4 is preferably semispherical, circular conical extending from the deepest part 4b of the recess 4 to the recess opening surface 4a, or circular truncated conical, particularly preferably semispherical. The semispherical means a shape having substantially half a sphere lacking and is not limited to a shape of a half of a sphere. Further, as shown in Fig. 3A, the shape of the recess 4 in a plan view is circular, but is not limited thereto and may, for example, be elliptic.

The recesses 4 at the bottom 3 shown in Figs. 3A and 3B are formed in a semisphere. The size of each recess 4 is described with reference to a case where the recesses 4 are in a semispherical shape. In each recess 4, the deepest part 4b means a position deepest from the flat region Sf. Further, in the thickness direction of the bottom 3, the recess opening surface 4a and the flat region Sf are at the same level.

The diameter Dh of the recess opening surface 4a is preferably at least 10 µm and at most 1,000 µm in view of dispersibility of inoculated cells. The diameter Dh of the recess opening surface 4a is more preferably at least 100 µm, further preferably at least 150 µm. The diameter Dh of the recess opening surface 4a is more preferably at most 800 µm, further preferably at most 700 µm. From the viewpoint of cell dispersibility, the diameter Dh is more preferably at least 100 µm and at most 800 µm, further preferably at least 150 µm and at most 700 µm.

The depth H of each recess 4 corresponding to the distance from the deepest part 4b of the recess 4 to the recess opening surface 4a is preferably at most the diameter Dh of the recess opening surface 4a in view of processing stability so as to stably form the recesses 4, in the bottom comprising a light-transmitting glass material. That is, the value obtained by diving the depth H of the recess 4 by the diameter Dh of the recess opening surface 4a (i.e. H/Dh) is preferably at most 1. H/Dh is more preferably at most 0.7. Further, H/Dh is preferably at least 0.25 in view of dispersibility of inoculated cells. The depth H of the recess 4 is preferably at least 50 µm and at most 500 µm in view of processing stability so as to stably form the recesses 4. The depth H of the recess 4 is more preferably at least 75 µm, further preferably at least 100 µm. The depth H of the recess 4 is more preferably at most 450 µm, further preferably at most 400 µm. From the viewpoint of the processing stability, the depth H is more preferably at least 75 µm and at most 450 µm, further preferably at least 100 µm and at most 400 µm.

The volume of each recess 4 is a volume of a space surrounded by the recess opening surface 4a and the inner surface of the recess 4, and corresponds to a volume of a space in which cells are cultured and spheroids are cultured and spheroids are prepared, in the cell culture container 10. The volume of the recess 4 is preferably from 2.0×10³ to 2.0×10⁹ µm³, more preferably from 2.0×10⁶ to 2.0×10⁹ µm³ in view of dispersibility of inoculated cells.

Considering efficiency in preparation of spheroids, it is preferred that the recesses 4 are arranged without a gap in the region S. From such a viewpoint, the value obtained by dividing the distance Dx between centers of the recess opening surfaces 4a of the adjacent recesses 4 by the diameter Dh of the recess opening surface 4a (that is, Dx/Dh) is preferably at least 1.0 and at most 1.2. When Dx/Dh is 1.0, the adjacent recesses 4 have such a structure that their recess opening surfaces 4a are in contact with each other. In view of easiness of production of the bottom 3, Dx/Dh is more preferably at least 1.05, and in view of efficiency of preparation of spheroids, Dx/Dh is more preferably at most 1.15.

The bottom 3 has a plurality of recesses 4 in the region S. The above numeral ranges of the diameter Dh of the recess opening surface 4a, the depth H of the recess 4 and Dx/Dh are ranges as average values of the plurality of recesses 4 present in the region S. The deviations of the diameters Dh of the recess opening surfaces 4a, the depths H of the recesses 4 and Dx/Dh with respect to the plurality of recesses 4 present in the region S are preferably within 10% of the average values, more preferably within 5%. The number of the recesses 4 provided in the region S is preferably the largest number of recesses which can be provided in the region S. The number of the recesses 4 in the region S depends on the area of the region S and the shape and the size of the recess opening surfaces 4a.

The proportion of the total area of the recess opening surfaces 4a of the recesses 4 in the region S, that is, the total area of the recesses 4 in a plan view, to the area of the region S in a plan view, that is, the whole area of the upper surface 3a which the opening 1 faces of the bottom 3, is preferably at least 40 %, more preferably at least 45%, particularly preferably at least 50%. Here, the area of the region S in a plan view is preferably from 1 to 50 mm², more preferably from 2 to 25 mm² in view of dispersibility of inoculated cells. In such a case, the number of the recesses 4 per unit area of the region S in a plan view is preferably from 2 to 50 recesses/mm², more preferably from 5 to 20 recesses/mm².

The shape of the region S is preferably rectangular including square or circular, in view of dispersibility of inoculated cells and efficiency of preparation of spheroids. According to the after-described embodiment of a cell culture container having a plurality of regions S, the shape of the region S is preferably rectangular, particularly preferably square, from the viewpoint of downsizing, production easiness, etc.

The material constituting the bottom 3 is a glass material having light-transmitting property. The glass material having light-transmitting property means that the spectral transmittance in a wavelength region of from 500 to 700 nm measured with respect to a glass plate having a thickness of 0.5 mm prepared from the glass material is at least 90%. The glass material is preferably such that when it constitutes the bottom 3, the spectral transmittance of the bottom 3 at a wavelength of 400 nm is at least 70%, more preferably at least 80%.

The glass material provides less autofluorescence as compared with a resin regardless of the composition. Accordingly, in fluorescence microscopic observation of spheroids obtained by cell culture, the background noises can be reduced, and high magnification observation is possible. The glass material may, for example, be specifically soda lime glass, aluminosilicate glass, quartz glass, alkali-free glass or borosilicate glass.

The glass material is preferably one with less autofluorescence, whereby high magnification and high precision fluorescence microscopic observation can be carried out. For example, the value obtained by dividing the fluorescence intensity at 584 nm when the glass material is excited by light at 532 nm by the fluorescence intensity at 584 nm when quartz glass is excited by light at 532 nm, measured by a 100 magnification objective lens by means of microscopic Raman spectroscopy (hereinafter sometimes referred to as "ratio of fluorescence intensity to quartz glass") is preferably at most 10, more preferably at most 9. Measurement by microscopic Raman spectroscopy is conducted, for example, by Almega (tradename) manufactured by Thermo Fisher Scientific. As the quartz glass, for example, AQ (tradename) manufactured by AGC Inc. may be used.

In observation of stained cells, blue light (excited at 435 nm, detected at 485 nm), green light (excited at 488 nm, detected at 520 nm) or red light (excited at 555 nm, detected at 584 nm) is used in many cases. Accordingly, as the glass material to be used for the bottom 3, it is considered that the lower the fluorescence intensity at 584 nm when excided by light at 532 nm, the larger the contrast difference with stained cells, whereby high magnification observation is possible, and accordingly such a glass material is suitable for fluorescence microscopic observation of cells.

Quartz glass is glass with the lowest fluorescence intensity value at 584 nm when excited by light at 532 nm. By a ratio of the fluorescence intensity to quartz glass being at most 10, for example, when such a glass material is used to prepare a glass substrate, the glass substrate is inoculated with stained cells (TIG-3 cells stained with Calcein-AM), and fluorescence microscopic observation is carried out with a 20 magnification objective lens, the stained cells can be visually confirmed. Of the resin material, the ratio of the fluorescence intensity to quartz glass is approximately from 50 to 500 regardless of the type.

As a glass material having the ratio of fluorescence intensity to quarts glass being at most 10, specifically, aluminosilicate glass, quartz glass and borosilicate glass may, for example, be mentioned, and glass having the following composition or commercial products are preferred.

As the aluminosilicate glass, glass having a composition comprising, as represented by mol% based on oxides, from 60 to 70% of SiO₂, from 2 to 20% of Al₂O₃, from 0 to 15% of B₂O₃, from 0 to 10% of Li₂O, from 0 to 20% of Na₂O, from 0 to 10% of K₂O, from 0 to 15% of MgO, from 0 to 10% of CaO, from 0 to 10% of SrO and from 0 to 10% of ZrO₂ is preferred. As a commercial product of the aluminosilicate glass, Dragontrail (manufactured by AGC Inc., registered trademark, ratio of fluorescence intensity to quartz glass: 8.5) may be mentioned.

The quartz glass is glass having a SiO₂ content of 100%. As a commercial product of the quartz glass, AQ (AGC Inc., tradename) may be mentioned. Any quartz glass has a constant value of the fluorescence intensity at 584 nm when excited by light at 532 nm.

As the borosilicate glass, preferred is glass having a composition comprising, as represented by mol% based on oxides, from 70 to 90% of SiO₂, from 0 to 5% of Al₂O₃, from 7 to 20% of B₂O₃, from 0 to 5% of Li₂O, from 0 to 10% of Na₂O, from 0 to 5% of K₂O and from 0 to 10% of ZrO₂. As a commercial product of the borosilicate glass, D263Teco (manufactured by SCHOTT AG, tradename, ratio of fluorescence intensity to quartz glass: 7.4) may be mentioned.

The bottom 3 may be produced, for example, by preparing a glass plate having flat principal planes and the same plate thickness as the thickness of the bottom 3, to be a base of the bottom 3 (hereinafter referred to as "base glass plate"), using a light-transmitting glass material, and providing a plurality of recesses at predetermined positions on one principal plane. As a method of providing the recesses, specifically, the following methods 1 and 2 may be mentioned.

### (Method 1)

To both principal planes of the base glass plate, a protective film such as a PET (polyethylene terephthalate) film is bonded. Then, a plurality of seed holes to be bases of recesses 4 are formed at predetermined intervals on a plane on which the recesses 4 are to be formed, by CO₂ laser. After the seed holes are formed, the protective films are peeled from the base glass plate, followed by annealing. The purpose of annealing is to remove residual stress by irradiation of glass with CO₂ laser, and the conditions are properly adjusted depending upon the glass composition.

Then, on the plane not having the seed holes formed of the base glass plate having the seed holes formed, a protective film is bonded, followed by shower etching by pouring an etching liquid containing hydrogen fluoride. After the etching, the protective film is peeled, whereby a bottom 3 having a plurality of recesses 4 formed on the upper surface 3a is obtained, as shown in Figs. 3A and 3B. Since isotropic etching is conducted in such a case, the depth H of each recess 4 is the depth of each seed hole, and the size of each recess 4 in a width direction, that is, the diameter Dh of each recess opening surface 4a depends on the etching time. Accordingly, in order to obtain recesses 4 having a desired size, the conditions of formation of the seed holes and the time of the shower etching are properly adjusted.

Instead of the shower etching, conventional etching by which the entire base glass plate having the seed holes formed thereon is dipped in an etching liquid. The etching liquid is an aqueous hydrogen fluoride solution, and may contain, as a component other than hydrogen fluoride, an acid other than hydrogen fluoride, such as sulfuric acid, hydrochloric acid, nitric acid or citric acid. By the liquid containing an acid other than hydrogen fluoride, local precipitation by reaction of the alkali component in the glass and hydrogen fluoride can be suppressed, whereby etching will proceed uniformly in plane.

### (Method 2)

On both principal planes of the base glass plate, a metal protective layer comprising a metal which can be etched with an etching liquid containing hydrogen fluoride is formed, and a photosensitive resin composition is applied on the metal protective layer. Then, the plane on which recesses 4 are to be formed is irradiated with active energy rays such as ultraviolet rays via a photomask which opens at a region other than the region on which the recesses 4 are to be formed, that is, the portion corresponding to the flat region Sf, whereby the photosensitive resin composition is cured only on the flat region Sf. Further, with respect to the plane on which the recesses 4 are not to be formed, the whole area is irradiated with active energy rays such as ultraviolet rays, whereby the photosensitive resin composition is cured. Then, the photosensitive resin composition in the non-exposed region corresponding to the region on which the recesses 4 are to be formed is removed by development.

Then, the above-obtained base glass plate having the metal protective layer and partly a cured film of the photosensitive resin composition is dipped in an etching liquid containing hydrogen fluoride for a predetermined time, and then dipped in a release agent to remove the metal protective layer and the cured film of the photosensitive resin composition. In such a manner, a base 3 having a plurality of recesses 4 formed on the upper surface 3a is obtained as shown in Figs. 3A and 3B. The etching liquid in the Method 2 is as defined for the Method 1. Further, when the base glass plate having the protective layer is dipped in the etching liquid, it may be shaken so as to shorten the treatment time.

In the cell culture container 10, the side walls 2 are provided around the periphery of the upper surface 3a of the bottom 3. The inner wall surfaces of the side walls 2 may be vertical to the flat region Sf of the upper surface 3a of the bottom 3, or may be tapered so that the opening 1 expands from the lower edge toward the upper edge. The outer wall surfaces of the side walls 2 are preferably vertical to the flat region Sf of the upper surface 3a of the bottom 3. The height of the side wall 2, that is, the depth of the opening 1, is preferably from 1 to 10 mm, more preferably from 2 to 10 mm in view of the required amount of the culture medium to be dispensed. The width of the side wall 2 is preferably from 0.5 to 2 mm, more preferably from 0.5 to 3 mm in view of the required amount of the culture medium to be dispensed.

Further, the side walls 2 may be bonded to the outside of the outer periphery of the bottom 3 in such a manner that the side surfaces of the bottom 3 and the lower region of the inner wall surfaces of the side walls 2 are bonded. In such a case, the depth of the opening 1 is preferably as mentioned above, and the height of the side wall 2 is preferably the sum of the depth of the opening 1 and the thickness of the bottom 3.

As the material constituting the side walls 2, an inorganic material such as glass or a resin may, for example, be mentioned, and a resin is preferred from the viewpoint of easiness of production. The resin may, for example, be an acrylic resin, polylactic acid, polyglycolic acid, a styrene resin, an acrylic/styrene copolymer resin, a polycarbonate resin, a polyester resin, a polyvinyl alcohol resin, an ethylene/vinyl alcohol copolymer resin, a thermoplastic elastomer, a vinyl chloride resin or a silicone resin, and is preferably a styrene resin in view of easiness of formation.

The method of bonding the side walls 2 and the bottom 3 is properly selected depending upon the material constituting the side walls 2. Since the bottom 3 is made of a glass material, in a case where the side walls 2 are made of a glass material, they may be integrally formed, or may be bonded e.g. by heat fusion. In a case where the side walls 2 are constituted by the resin, preferably, the side walls 2 and the bottom 3 are bonded via an adhesive layer properly selected depending upon the type of the resin.

The surface layer 5 has a property to inhibit cell adhesion. In the cell culture container 10 shown in Fig. 2, the surface layer 5 is provided on the whole region S including the inner surface of the recesses 4 and the flat region Sf, however, in the cell culture container of the present invention, the surface layer should be provided at least only at the inner surface of the recesses 4. In the cell culture container 10, cell culture is conducted in microspaces M each surrounded by the surface of the surface layer 5, whereby cells are efficiently aggregated one another without being bonded to the surface layer 5 to form spheroids, and the spheroids are easily taken out from the microspaces M. Further, by the flat region Sf also having the surface layer 5, the spheroids can easily be taken out from the cell culture container 10. The surface layer 5 may be formed on the inner wall surface of the side walls 2.

The surface layer 5 has a property to inhibit cell adhesion preferably by having a biocompatible group. As the biocompatible group, a known organic group such as a polyoxyalkylene group or a phosphorylcholine group may be used. Specifically, the biocompatible group which the surface layer 5 has preferably comprises at least one type selected from the group consisting of a group represented by the following formula 1, a group represented by the following formula 2 and a group represented by the following formula 3:

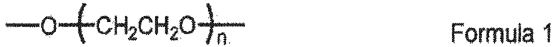

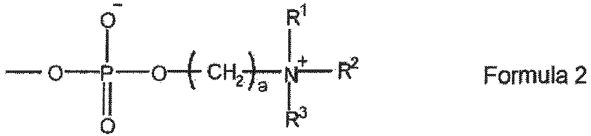

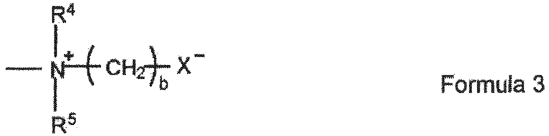

In the formula 1, n is an integer of from 1 to 300, and from 50 to 100 mol% of the groups represented by the formula 1 are groups represented by the formula 1 in groups represented by the following formula 4. n in the formula 4 is an integer of from 1 to 300, and R⁶ is a hydrogen atom or a C₁₋₅ alkyl group.

In the formula 2, R¹ to R³ are each independently a C₁₋₅ alkyl group, and a is an integer of from 1 to 5.

In the formula 3, R⁴ and R⁵ are each independently a C₁₋₅ alkyl group, X- is a group represented by the following formula 3-1 or a group represented by the following formula 3-2, and b is an integer of from 1 to 5.

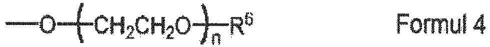

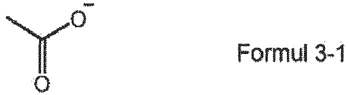

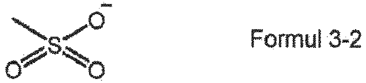

In this specification, the alkyl group may be linear, branched or cyclic, or may be a combination thereof.

Hereinafter, the group 1 (provided that from 50 to 100 mol% thereof is the group 1 in the group 4) will be referred to as "group 1(4)". The biocompatible group which the surface layer 5 has may contain any one type of the group 1(4), the group 2 and the group 3, or may contain two or more types thereof. The biocompatible group is preferably the group 1(4). The surface layer 5 may have a biocompatible group other than the group 1(4), the group 2 and the group 3 as the case requires, however, the biocompatible group which the surface layer 5 has preferably comprises only at least one type selected from the group 1(4), the group 2 and the group 3.

It is preferred that a covalent bond is present between the surface layer 5 and the bottom 3. By the surface layer 5 and the bottom 3 being bonded via the covalent bond, the surface layer 5 has sufficient durability. Further, it is preferred that the amount of the total organic carbon (TOC) eluted from the surface layer 5 into water per unit area 1 cm² of the surface layer 5 when dipped in water at 40°C for 7 days (hereinafter sometimes referred to as "TOC elution amount") is preferably at most 1 mg/L. The TOC elution amount is, in other words, the mass [mg] of the TOC eluted into water when the surface layer with an area of 1 cm² is dipped in 1 L of water at 40°C for 7 days. If the constituents are eluted from the surface layer 5, they may influence cell culture or microscopic observation, and accordingly the TOC elution amount is preferably at most 1 mg/L. The TOC elution amount is more preferably at most 0.5 mg/L, further preferably at most 0.3 mg/L.

TOC is the total amount of organic substances represented by the amount of carbon. In this specification, the TOC elution amount of the surface layer may be measured, specifically, as follows. The surface layer is dipped in a predetermined amount of water at 40°C for 7 days, and the TOC concentration [mg/L] of the water used for the treatment is measured. The water to be used for dipping is distilled water or deionized water. The above-obtained TOC concentration is divided by the area (unit: cm²) of the surface layer dipped, to obtain the TOC elution amount [mg/L]. The TOC concentration in water may be measured by a conventional TOC analyzer, for example, TNC-6000 (manufactured by TORAY ENGINEERING Co., Ltd.).

As a sample of the surface layer to be used to measure the TOC elution amount, a surface layer itself prepared on and then peeled from a releasable substrate may be used, or a surface layer-provided substrate having a surface layer formed on a substrate with a TOC elution amount of 0 [mg/L] under the above conditions (40°C, 7 days) may be used.

The surface layer 5 having a covalent bond with the bottom 3 and having a property to inhibit cell adhesion, specifically, having the biocompatible group, is preferably a surface layer 5 comprising a cured product of a composition containing a compound having a group capable of forming a covalent bond with the bottom 3 made of the glass material, and a biocompatible group. The group capable of forming a covalent bond with the bottom 3 made of the glass material, of the compound, is preferably a hydrolyzable silyl group, more preferably an alkoxysilyl group.

Further, the compound is preferably a compound having a biocompatible group which is at least one type selected from the group 1(4), the group 2 and the group 3 and an alkoxysilyl group (hereinafter referred to as compound (X)). Further, the composition to be used for forming the surface layer 5 is preferably a composition containing the compound (X), having a content of the biocompatible group in the solid content of the composition of from 25 to 83 mass%, and having a content of the alkoxysilyl group of from 2 to 70 mass% (hereinafter referred to as composition (Y)). Hereinafter, the composition for forming the surface layer 5 will be described with reference to the composition (Y), however, the composition for forming the surface layer 5 is not limited thereto so long as the obtainable surface layer is within the range of the present invention.

The solid content in the composition means a residue after the composition is vacuum dried at 80°C for 3 hours to remove the volatile components. The cured product of the composition is a cured product of the solid content. Further, in the following description, unless otherwise specified, the "biocompatible group" is a biocompatible group comprising at least one type selected from the group consisting of the group represented by the formula 1, the group represented by the formula 2 and the group represented by the formula 3.

Here, the surface layer 5 comprising the cured product of the composition (Y) containing the compound (X) means that the surface layer 5 contains at least a cured product of a component capable of hydrolytic condensation including the compound (X). When the composition (Y) is cured, the compound (X), which has an alkoxysilyl group, is hydrolyzed to form a silanol group (Si-OH). Then, such silanol groups undergo dehydration condensation to form a siloxane bond (Si-O-Si) to form a cured product. On that occasion, also when the composition (Y) contains a hydrolysable silyl group-containing component other than the compound (X), preferably an alkoxysilyl group-containing component, similarly, the component and the compound (X) form a siloxane bond.

In a case where the composition (Y) is cured on the surface of the bottom 3, the silanol group formed by hydrolysis of the hydrolysable silyl group-containing component in the composition (Y) containing the compound (X), undergoes dehydration condensation with the hydroxy group (glass material-OH) on the surface of the bottom 3, at the same time as formation of the Si-O-Si bond, to form a covalent bond (glass material-O-Si), whereby the obtainable surface layer 5 is strongly bonded to the surface of the bottom 3 and thereby has high durability, for example, water resistance.

Since the composition (Y) has a content of the biocompatible group of at least 25 mass%, the obtainable surface layer 5 has a sufficient amount of the biocompatible group and can more effectively inhibit cell adhesion. Since the content of the biocompatible group is at most 83 mass%, water resistance can be imparted. The content of the biocompatible group in the solid content in the composition (Y) is preferably from 30 to 83 mass%, more preferably from 40 to 83 mas%.

Since the composition (Y) has a content of the alkoxysilyl group of at least 2%, the alkoxysilyl groups form a sufficient amount of covalent bonds with the surface of the bottom 3 when the composition (Y) is cued, and the obtainable surface layer 5 is excellent in durability, for example, water resistance. Since the content of the alkoxysilyl group is at most 70 mass%, a sufficient amount of the biocompatible groups can be introduced. The content of the alkoxysilyl group in the solid content in the composition (Y) is preferably from 2 to 40 mass%, more preferably from 2 to 30 mass%.

For example, the cell culture container 10 may have a silicon oxide layer between the surface layer 5 and the bottom 3 so as to make the covalent bond between the surface layer 5 and the bottom 3 more firm. The silicon oxide layer is preferably a silicon oxide layer having a thickness of from about 1 to about 2 nm obtained by deposition. Since the surface layer 5 and the silicon oxide layer, and the silicon oxide layer and the bottom 3, are respectively bonded via a covalent bond, the case where the cell culture container 10 has the silicon oxide layer between the surface layer 5 and the bottom 3 is included in the category that "the surface layer 5 has a covalent bond with the bottom 3".

The alkoxysilyl group which the compound (X) has may, for example, be a group represented by the formula 5.

-Si(R⁷)₃₋ₜ(OR⁸)ₜ Formula 5

In the formula 5, R⁷ is a C₁₋₁₈ alkyl group, R⁸ is a C₁₋₁₈ alkyl group, and t is an integer of from 1 to 3. In a case where more than one R⁷ or OR⁸ are present, such R⁷ or R⁸ may be the same or different. From the production viewpoint, R⁷ or R⁸ are preferably the same.

From the viewpoint of the adhesion between the bottom 3 and the surface layer 5, t is preferably at least 2, more preferably 3. From the viewpoint of steric hinderance at the time of condensation reaction, R⁷ is preferably a C₁₋₇ alkyl group, more preferably a methyl group or an ethyl group. From the viewpoint of the hydrolytic reaction rate and volatility of by-products at the time of the hydrolysis reaction, R⁸ is preferably a C₁₋₆ alkyl group, more preferably a methyl group or an ethyl group.

The compound (X) may, for example, be a compound (X1) comprising a polyoxyethylene chain as the main chain and having an alkoxysilyl group at the terminal or in a side chain, or a compound (X2) comprising a hydrocarbon chain having ethylenic double bonds polymerized as the main chain and having the biocompatible group and the alkoxysilyl group in a side chain, which satisfies the requirements as the compound (X).

The compound (X1) may be obtained, for example, by introducing the alkoxysilyl group to a polyoxyethylene polyol or a polyoxyethylene alkyl ether having at least one hydroxy group (provided that the alkyl has from 1 to 5 carbon atoms) via the hydroxy group or the linking group which such a compound has. More specifically, the compound (X1) is obtained, for example, by reacting a polyoxyalkylene polyol having a polyoxyethylene chain or a polyoxyalkylene alkyl ether having a polyoxyethylene chain and having at least an hydroxy group (provided that the alkyl has from 1 to 5 carbon atoms) with a silane compound having a group reactive with the hydroxy group and having the alkoxysilyl group (hereinafter sometimes referred to as silane compound (S)) at a predetermined proportion.

The polyoxyalkylene polyol to be used may, for example, be a compound obtained by subjecting an alkylene monoepoxide at least including ethylene oxide to ring-opening addition polymerization to a polyol having a relatively low molecular weight, such as an alkane polyol, an etheric oxygen atom-containing polyol or a sugar alcohol. The oxyalkylene group in the polyoxyalkylene polyol may, for example, be an oxyethylene group, an oxypropylene group, an oxy-1,2-butylene group, an oxy-2,3-butylene group or an oxyisobutylene group.

The polyoxyalkylene alkyl ether to be used may be a compound having some of hydroxy groups in such a polyoxyalkylene polyol ether-bonded with a C₁₋₅ aliphatic alcohol. In the following description, unless otherwise specified, the "polyoxyalkylene alkyl ether" means a polyoxyalkylene alkyl ether having at least one hydroxy group (provided that the alkyl has from 1 to 5 carbon atoms). The same applies to a case where the "oxyalkylene" is "oxyethylene".

The oxyalkylene groups of the polyoxyalkylene polyol and the polyoxyalkylene alkyl ether may consist solely of oxyethylene groups or may comprise a combination of oxyethylene groups and other oxyalkylene groups. In view of easiness of molecular design of the compound (X1), preferred is a polyoxyethylene polyol or polyoxyethylene alkyl ether having only oxyethylene groups. Hereinafter, the polyoxyethylene polyol and the polyoxyethylene alkyl ether may sometimes be generally referred to as a polyoxyethylene polyol or the like.

That is, the compound (X1) is preferably a reaction product of the polyoxyethylene polyol or the like and the silane compound (S). The number of hydroxy groups in the polyoxyethylene polyol or the like may be from 1 to 6, and in view of easiness of molecular design of the compound (X1), preferably from 1 to 4, particularly preferably from 1 to 3. The polyoxyethylene polyol or the like may, for example, be specifically polyoxyethylene glycol, polyoxyethylene glyceryl ether, trimethylolpropane trioxyethylene ether, pentaerythritol polyoxyethylene ether, dipentaerythritol polyoxyethylene ether or polyoxyethylene glycol monoalkyl ether (provided that the alkyl has from 1 to 5 carbon atoms).

For example, in a case where the polyoxyethylene polyol or the like is polyoxyethylene glycol having two hydroxy groups, the compound (X1) may be compound (X11) represented by the reference symbol (X11), obtained by reaction of the polyoxyethylene glycol and silane compound (S1) represented by R⁹-Q¹¹-Si(R⁷)₃₋ₜ(OR⁸)ₜ as shown in the following formula.

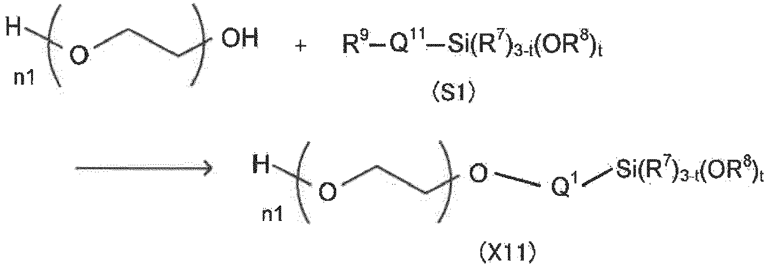

In the above reaction formula, n1 in polyoxyethylene glycol is an integer of from 1 to 300, preferably from 2 to 100, more preferably from 4 to 20. In the silane compound (S1), R⁷, R⁸ and t are as defined for the formula 5 including the preferred embodiments. In the silane compound (S1), R⁹ is a group reactive with a hydroxy group and may be a hydroxy group, a carboxy group, an isocyanate group or an epoxy group. Q¹¹ is a C₂₋₂₀ bivalent hydrocarbon group which may have an etheric oxygen atom between carbon atoms and in which the hydrogen atom may be substituted by a halogen atom such as a chlorine atom or a fluorine atom or a hydroxy group. In a case where the hydrogen atom is substituted by a hydroxy group, the number of the substituting hydroxy group is preferably from 1 to 5.

In the formula (X11), Q¹ is a residue having R⁹-Q¹¹ in the silane compound (S1) reacted with the hydroxy group of the polyoxyethylene glycol, and is represented by R^{9'}-Q¹¹ (R^{9'} is on the side bonded to O, and Q¹¹ is on the side bonded to the alkoxysilyl group). R^{9'} corresponds to R⁹ and may be a single bond, -C(=O)-, -C(=O)NH-,-C(=O)N(CH₃)-, -C(=O)N(C₆H₅)- or -CH₂CH(-OH)CH₂O-. Hereinafter, -C(=O)N··· will be represented as -CON···. For example, -C(=O)NH- is represented as -CONH-.

Q¹ may, for example, be preferably -(CH₂)ₖ-, -CONH(CH₂)ₖ-, -(CF₂)ₖ- (k is an integer of from 2 to 4), -CH₂OC₃H₆-, -CF₂OC₃H₆-. Among them, more preferred is any one selected from -CONHC₃H₆-, -CONHC₂H₄-, -CH₂OC₃H₆-, -CF₂OC₃H₆-, -C₂H₄-,-C₃H₆-, and -C₂F₄-. Further preferred is -CONHC₃H₆-, -CONHC₂H₄-, -C₂H₄- or -C₃H₆-.

Otherwise, the polyoxyethylene glycol may be reacted with allyl chloride under basic conditions, followed by silane modification by hydrosilylation to obtain compound (X11).

The proportion of the group 1 in the compound (X11) being the group 1 in the group 4 is 100 mol%. That is, the group 1 in the compound (X11) is entirely the group 1 contained in the group 4. The content of the biocompatible group in the compound (X11) is the mass% of ₙ₁(OCH₂CH₂)-O in the formula (X11), and the content of the alkoxysilyl group is mass% of -Si(R⁷)₃₋ₜ(OR⁸)ₜ in the formula (X11). The contents of the biocompatible group and the alkoxysily group in the compound (X11) are properly adjusted depending upon the solid content composition of the composition (Y). The content of the biocompatible group in the compound (X11) is, for example, preferably from 10 to 90 mass%, more preferably from 25 to 83 mass%, further preferably from 40 to 83 mass%, particularly preferably from 60 to 83 mass%. The content of the alkoxysilyl group in the compound (X11) is preferably from 1 to 70 mass%, more preferably from 2 to 70 mass%, further preferably from 2 to 45 mass%, particularly preferably from 10 to 30 mass%.

Further, the compound (X11) in which the terminal hydrogen atom is substituted by R⁶ other than the hydrogen atom may also be used as the compound (X1). That is, a compound obtained by using a polyoxyethylene glycol monoalkyl ether (wherein the alkyl is R⁶) instead of the polyoxyethylene glycol having two hydroxy groups in the above reaction formula may also be used as the compound (X1). In such a case, R⁶ is preferably a methyl group or an ethyl group, more preferably a methyl group.

For example, in a case where the polyoxyethylene polyol is polyoxyethylene glyceryl ether having three hydroxy groups, the compound (X1) may be compound (X12) represented by the reference symbol (X12), obtained by reaction of the polyoxyethylene glyceryl ether and the silane compound (S1) represented by R⁹-Q¹¹-Si(R⁷)₃₋ₜ(OR⁸)ₜ as shown in the following formula.

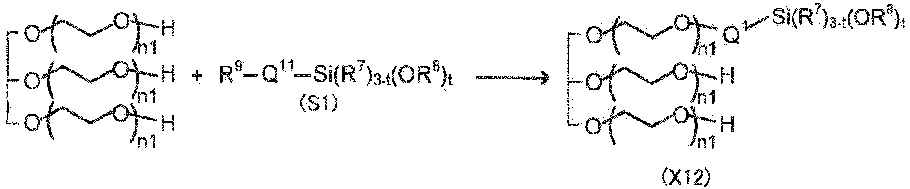

In the above reaction formula, n1 in the polyoxyethylene glyceryl ether is as defined for n1 in the polyoxyethylene glycol including the preferred embodiment. The silane compound (S1) is as defined above. In the compound (X12), Q¹ is as defined for Q¹ in the compound (X11) including the preferred embodiment.

The proportion of the group 1 in the compound (X12) being the group 1 in the group 4 is 67 mol%. The content of the biocompatible group in the compound (X12) is the total mass% of O-(CH₂CH₂O)ₙ₁- and O-(CH₂CH₂O)ₙ₁-H in the formula (X12) and is adjusted to from 25 to 83 mass%. The content of the biocompatible group and the content of the alkoxysilyl group in the compound (X12) are as defined in the case of the compound (X11) including the preferred embodiments.

The compound (X12) in which the terminal hydrogen atom in O-(CH₂CH₂O)ₙ₁-H is substituted by R⁶ other than the hydrogen atom may also be used as the compound (X1). R⁶ in such a case is preferably a methyl group.

The content of structures other than the biocompatible group and the alkoxysilyl group in the compound (X1) is, with a view to satisfying both cell non-adhesiveness and durability, particularly water resistance, of the surface layer, preferably from 10 to 50 mass%, more preferably from 20 to 30 mass%. The weight average molecular weight of the compound (X1) is, from the viewpoint of availability of raw materials, preferably from 100 to 10,000, more preferably from 500 to 2,000. The weight average molecular weight (hereinafter sometimes referred to as "Mw") of the compound (X1) is calculated by size exclusion chromatography.

The compound (X1) was explained using as the polyoxyethylene polyol or the like polyoxyethylene glycol and polyoxyethylene glyceryl ether as examples. With respect to other polyoxyethylene polyol or the like, in the same manner, the compound (X1) can be produced by properly adjusting the proportion of the group 1 being the group 1 in the group 4, the content of the biocompatible group, the content of the alkoxysilyl group, etc. to desired proportions.

The compound (X1) may further be a partially hydrolyzed condensate. In a case where the compound (X1) is a partially hydrolyzed condensate, as described hereinafter, the degree of condensation may be properly adjusted so as to achieve a viscosity to such an extent that formation of the surface layer 5 on the surface of the bottom 3 is not impaired. From such a viewpoint of the viscosity, Mw of the partially hydrolyzed condensate is preferably from 1,000 to 1,000,000, more preferably from 1,000 to 100,000. A preferred range of Mw is the same with respect to the following partially hydrolyzed co-condensate. The content (mass%) of the alkoxysilyl group in the partially hydrolyzed condensate is regarded as equal to the content (mass%) of the alkoxysilyl group in the raw material silane compound. In the partially hydrolyzed co-condensate, the content (mass%) of the alkoxysilyl group may be calculated from the proportion of the raw material silane compound mixed.

The compound (X1) may be a partially hydrolyzed co-condensate obtained by subjecting two or more types of the compounds (X1) to partial hydrolytic co-condensation so as to achieve the desired proportions of the biocompatible group and the alkoxysilyl group. The compound (X1) may also be a partially hydrolyzed co-condensate obtained by subjecting the compound (X1) and an alkoxysilane compound having no biocompatible group to partial hydrolytic co-condensation so that the obtainable partially hydrolyzed co-condensate contains the biocompatible group and the alkoxysilyl group at desired proportions as the compound (X).

As the alkoxysilane compound having no biocompatible group, an alkoxysilane compound of the following formula 6 may be mentioned.

Si(R²⁰)₄₋ₚ(OR²¹)ₚ Formula 6

In the formula 6, R²⁰ is a monovalent organic group having no polyoxyethylene chain, R²¹ is a C₁₋₁₈ alkyl group, and p is an integer of from 1 to 4. In a case where more than one R²⁰ or more than OR²¹ are present, such R²⁰ and R²¹ may be respectively the same or different. From the viewpoint of production, R²⁰ and R²¹ are respectively preferably the same.

As R²⁰, specifically, a C₁₋₁₈ alkyl group may be mentioned, and a methyl group is preferred from the viewpoint of steric hinderance at the time of the condensation reaction.

From the viewpoint of adhesion between the bottom 3 and the surface layer 5, p is preferably at least 2, more preferably 3 or 4, particularly preferably 4. From the viewpoint of the hydrolysis reaction rate and volatility of by-products at the time of the hydrolysis reaction, R²¹ is preferably a C₁₋₆ alkyl group, more preferably a methyl group or an ethyl group.

The compound (X2) may, for example, be a (meth)acrylate copolymer obtained by copolymerizing monomers essentially including a (meth)acrylate having a biocompatible group and a (meth)acrylate having an alkoxysilyl group and optionally including other (meth)acrylate. In such a case, the contents of the respective (meth)acrylates as the raw material monomers are adjusted so that the obtainable (meth)acrylate copolymer has the biocompatible groups and the alkoxysilyl groups at predetermined proportions as the compound (X).

As the (meth)acrylate copolymer, for example, copolymer (X21) represented by the following formula (X21) may be mentioned.

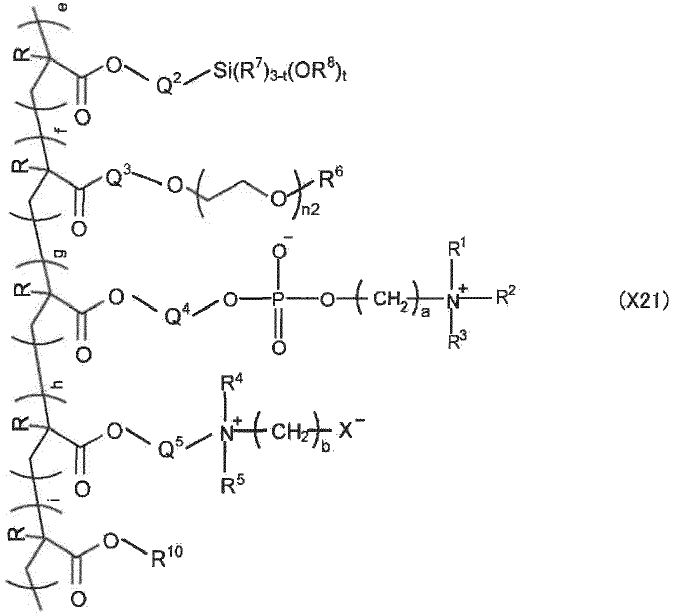

In the formula (X21), R¹ to R⁶, X⁻, a and b are as defined for the formulae 1 to 4. R¹ to R³ are preferably each independently a methyl group, and R⁴ and R⁵ are preferably each independently a methyl group. R⁶ is preferably a methyl group or a hydrogen atom. a and b are preferably each independently 2.

n2 is an integer of from 1 to 300, preferably from 1 to 100, more preferably from 1 to 20. R⁷, R⁸ and t are as defined for the formula 5 including the preferred embodiments.

R is a hydrogen atom or a methyl group independently of the respective units. R¹⁰ is a hydrogen atom or a monovalent organic group having no biocompatible group nor alkoxysilyl group. R¹⁰ is preferably a hydrogen atom or a C₁₋₁₀₀ alkyl group, more preferably a C₁₋₂₀ alkyl group.

The copolymer (X21) may be a random copolymer or a block copolymer.

Q², Q⁴ and Q⁵ are a C₂₋₁₀ bivalent hydrocarbon group which may have an etheric oxygen atom between carbon atoms and in which the hydrogen atom may be substituted by a halogen atom such as a chlorine atom or a fluorine atom or a hydroxy group.

Q² is preferably -C₂H₄-, -C₃H₆- or -C₄H₈-, more preferably -C₃H₆- or -C₄H₈-, further preferably -C₃H₆-.

Q⁴ and Q⁵ are preferably each independently -C₂H₄-, -C₃H₆- or -C₄H₈-, more preferably -C₂H₄- or -C₃H₆-, further preferably -C₂H₄-.

Q³ is a single bond or -O-Q⁶-, and Q⁶ is as defined for Q². Q³ is preferably a single bond.

In the copolymer (X21), e represents the number of units having an alkoxysilyl group (hereinafter referred to as units (A)) based on the number of all units of the copolymer being 100. Likewise, f, g, h and i respectively represent the numbers of units having the group 1(4) (hereinafter referred to as units (B1), units having the group 2 (hereinafter referred to as units (B2)), units having the group 3 (hereinafter referred to as units (B3)) and units represented by -(C-C(R)(C(=O)OR¹⁰))ᵢ- (hereinafter referred to as units (C)) based on the number of all units of the copolymer being 100. Hereinafter, -C(=O)O··· will be represented as -COO···.

By adjusting the proportions of e to i in the formula (X21), the contents of the biocompatible group and the alkoxysilyl group (-Si(R⁷)₃₋ₜ(OR⁸)ₜ) in the copolymer (X21) can be adjusted. The proportions of e to i in the copolymer (X21) are properly adjusted depending upon the solid content composition of the composition (Y). The content of the biocompatible group in the copolymer (X21) is, for example, preferably from 20 to 90 mass%, more preferably from 25 to 83 mass%, further preferably from 30 to 83 mass%, particularly preferably from 40 to 83 mass%. The content of the alkoxysilyl group in the copolymer (X21) is preferably from 1 to 70 mass%, more preferably from 2 to 70 mass%, further preferably from 2 to 25 mass%, particularly preferably from 2 to 15 mass%.

The copolymer (X21) is preferably a copolymer constituted solely by the units (A) and the units (B1). Here, (meth)acrylates to be raw materials of the units (A), the units (B1), the units (B2), the units (B3) and the units (C) will be respectively referred to as (meth)acrylate (A), (meth)acrylate (B1), (meth)acrylate (B2), (meth)acrylate (B3) and (meth)acrylate (C). Further, the (meth)acrylate (B1), the (meth)acrylate (B2) and the (meth)acrylate (B3) will be generally referred to as (meth)acrylate (B). In the following description regarding the (meth)acrylates, all reference symbols are the same as those in the copolymer (X21).

The (meth)acrylate (A) is CH₂=CR-COO-Q²-Si(R⁷)₃₋ₜ(OR⁸)ₜ, preferably CH₂=CR-COO-Q²-Si(OR⁸)₃, particularly preferably CH₂=CR-COO-(CH₂)₃-Si(OCH₃)₃ or CH₂=CR-COO-(CH₂)₃-Si(OC₂H₅)₃.

The (meth)acrylate (B1) is CH₂=CR-CO-Q³-O-(CH₂CH₂O)ₙ₂-R⁶, preferably CH₂=CR-COO-(CH₂CH₂O)ₙ₂-R⁶ (wherein n2 is from 1 to 300, and R⁶ is H or CH₃). n2 is more preferably from 1 to 20.

The (meth)acrylate (B2) is CH₂=CR-COO-Q⁴-(PO₄⁻)-(CH₂)ₐ-N⁺R¹R²R³, preferably CH₂=CR-COO-(CH₂)₂-(PO₄⁻)-(CH₂)₂-N⁺(CH₃)₃.

The (meth)acrylate (B3) is CH₂=CR-COO-Q⁵-N⁺R⁴R⁵-(CH₂)_{b}-X⁻, preferably CH₂=CR-COO-(CH₂)₂-N⁺(CH₃)₂-CH₂-COO⁻.

The (meth)acrylate (C) is CH₂=CR-COO-R¹⁰, and may, for example, be methyl methacrylate, butyl methacrylate or dodecyl methacrylate.

The copolymer (X21) may be obtained, for example, by copolymerizing the raw material (meth)acrylates so that e to i satisfy the above predetermined proportions, in the presence of a polymerization initiator, by a conventional method such as solution polymerization, bulk polymerization, suspension polymerization or emulsion polymerization.

In the compound (X2), the content of structures other than the biocompatible group and the alkoxysilyl group is, with a view to satisfying both cell non-adhesiveness and durability, particularly water resistance, of the surface layer, preferably from 15 to 55 mass%, more preferably from 15 to 40 mass%. Mw of the compound (X2) is, from the viewpoint of easiness of production, preferably from 1,000 to 1,000,000, more preferably from 20,000 to 100,000. Mw of the compound (X2) may be calculated by size exclusion chromatography.

The compound (X2) may further be a partially hydrolyzed condensate. In a case where the compound (X2) is a partially hydrolyzed condensate, as described hereinafter, the degree of condensation may be properly adjusted so as to achieve a viscosity to such an extent that formation of the surface layer 5 on the surface of the bottom 3 is not impaired. From such a viewpoint of the viscosity, Mw of the partially hydrolyzed condensate is preferably from 2,000 to 2,000,000, more preferably from 30,000 to 300,000. A preferred range of Mw is the same with respect to the following partially hydrolyzed condensate.

The compound (X2) may be a partially hydrolyzed co-condensate obtained by subjecting two or more types of the compounds (X2) to partial hydrolytic co-condensation so as to achieve the desired proportions of the biocompatible group and the alkoxysilyl group. The compound (X2) may also be a partially hydrolyzed co-condensate obtained by subjecting the compound (X2) and an alkoxysilane compound having no biocompatible group to partial hydrolytic co-condensation so that the obtainable partially hydrolyzed co-condensate contains the biocompatible group and the alkoxysilyl group at desired proportions as the compound (X).

The composition (Y) may contain one type of the compound (X) alone or may contain two or more types. When two or more type of the compounds (X) are used, the composition (Y) preferably comprises two or more types of only the compounds (X1) or comprises two or more types of only the compounds (X2). In a case where the solid content in the composition (Y) comprises only the compound (X), the compound (X) is selected so that the content of the biocompatible group and the content of the alkoxysilyl group are within the above predetermined ranges. The proportion of the compound (X) in the solid content in the composition (Y) is, for example, preferably from 25 to 100 mass%, more preferably from 50 to 100 mass%, further preferably from 75 to 100 mass%.

The composition (Y) may contain a component other than the compound (X). Such other component may be other solid content other than the compound (X) contained as the solid content in the surface layer 5. In a case where the surface layer is formed by dry coating, the composition (Y) contains only solid contents. On the other hand, in a case where the surface layer is formed by wet coating, the composition (Y) may further contain, as other component, a liquid medium to be removed when the surface layer is formed.

Other solid content may be a curable component like the compound (X), or may be a non-curable component. As other solid content, impurities which could not be removed among raw materials used in the process for producing the compound (X) and by-products, a functional additive, a catalyst, etc. may be mentioned. The functional additive may, for example, be an ultraviolet absorber, a light stabilizer, an antioxidant or a levelling agent.

Other solid content is preferably such a solid content that the obtainable surface layer 5 satisfies the above range of the TOC elution amount. Other solid content is, specifically, preferably a component capable of hydrolytic condensation with the compound (X), more preferably a hydrolyzable silyl group-containing component other than the compound (X), further an alkoxysilyl group-containing component. Particularly preferably, the composition (Y) contains no solid content other than the compound (X). In a case where the composition (Y) contains the compound (X) alone as the solid content, the compound (X) preferably contains the biocompatible group in a proportion of from 25 to 83 mass% and the alkoxysilyl group in a proportion of from 2 to 70 mass%.

As the catalyst, a known catalyst to be used for hydrolytic condensation reaction of an alkoxysilyl group may be used without any particular restriction. The catalyst may, for example, be specifically an acid such as hydrochloric acid, nitric acid, acetic acid, sulfuric acid, phosphoric acid, or sulfonic acid, for example, methanesulfonic acid or p-toluenesulfonic acid, a base such as sodium hydroxide, potassium hydroxide or ammonia, or an aluminum-based or titanium-based metal catalyst.

In a case where the compound (X1) is used as the compound (X), as other solid content, an alkoxysilane compound having no biocompatible group and/or its partially hydrolyzed condensate may be used. The alkoxysilane compound having no biocompatible group is preferably the compound 6. In a case where the alkoxysilane compound having no biocompatible group is formed into a partially hydrolyzed condensate, its Mw is preferably from 100 to 100,000, more preferably from 100 to 10,000.

In a case where the composition (Y) comprises as the solid content the compound (X1) and the alkoxysilane compound having no biocompatible group, preferably the content of the biocompatible group is from 25 to 83 mass% and the content of the alkoxysilyl group is from 2 to 70 mass%, to the total amount of the compound (X1) and the alkoxysilane compound having no biocompatible group. That is, it is preferred that the composition (Y) contains no compound other than the compound having the biocompatible group and/or alkoxysilyl group as the solid content. In such a case, the proportion of the alkoxysilane compound having no biocompatible group per 100 parts by mass of the compound (X1) is preferably from 50 to 200 parts by mass, more preferably from 50 to 100 parts by mass.

In a case where the compound (X) is used as the compound (X1), the content of solid content other than the compound (X1), the alkoxysilane compound having no biocompatible group and the catalyst, to the total solid content, is preferably at most 40 mass% in total, more preferably at most 20 mass%, and most preferably no such other solid content is contained.

Also in a case where the compound (X2) is used as the compound (X), an alkoxysilane compound other than the compound (X2) may be used as the case requires. In a case where the compound (X2) is used as the compound (X), the content of solid content other than the compound (X2) and the catalyst in the total solid content is preferably at most 40 mass% in total, more preferably at most 20 mass%, and most preferably no such other solid content is contained.

In a case where the surface layer 5 is formed by wet coating, the liquid medium contained in the composition (Y) may be any medium so long as the solid content including the compound (X) is uniformly dissolved or dispersed, and may be properly selected from among known liquid media. Since the liquid medium should be finally removed in formation of the surface layer, its boiling point is preferably within a range of from 60 to 160°C, more preferably from 60 to 120°C.

As the liquid medium, specifically, an alcohol, an ether, a ketone, an acetic acid ester or the like is preferred. As the liquid medium which satisfies the above boiling point, specifically, isopropyl alcohol (IPA), ethanol, propylene glycol monomethyl ether or 2-butanone may, for example, be mentioned. They may be used alone or in combination of two or more.

The liquid medium may contain water for the hydrolysis reaction of the hydrolyzable silyl group-containing component including the compound (X), but preferably contains no water from the viewpoint of storage stability. However, even in a case where the liquid medium contains no water, the hydrolyzable silyl group-containing component including the compound (X) may undergo hydrolysis reaction by moisture in the air, and thus presence of water in the liquid medium is not essential.

In a case where the composition (Y) contains the liquid medium, the solid content concentration in the composition (Y) is preferably from 0.1 to 50 mass%, more preferably from 1 to 30 mass%, further preferably from 1 to 15 mass%. When the solid content concentration is within the above range, the film thickness of the surface layer formed by wet coating using the composition (Y) is likely to be within a preferred range in which the algae resistance and its retention are sufficiently achieved. The solid content concentration of the composition (Y) may be calculated from the mass after the composition (Y) is vacuum dried at 80°C for 3 hours and the mass of the composition (Y) before heating. Or it may be calculated from the total solid content blended at the time of producing the composition (Y) and the amount of the liquid medium.

In a case where the composition (Y) contains the liquid medium, it preferably contains the liquid medium in an amount of from 50 to 99.5 mass%, more preferably from 65 to 99 mass%, further preferably from 70 to 99 mass%.

The method for producing the composition (Y) is not particularly limited. The solid content including the compound (X), or in a case where the liquid medium is further contained, the solid content and the liquid medium are mixed so as to achieve the above contents. The composition (Y), as described above, contains the compound (X), has a content of the biocompatible group of from 25 to 83 mass% and a content of the alkoxysilyl group of from 2 to 70 mass% in the solid content, and accordingly the surface layer 5 comprising a cured product of the composition (Y) formed on the surface of the bottom 3 is excellent in ability to inhibit cell adhesion and is excellent in durability, particularly water resistance as well.

The thickness of the surface layer 5 is preferably from 0.5 to 20 nm, particularly preferably from 0.5 to 10 nm. When the thickness of the surface layer is at least the lower limit value of the above range, ability to inhibit cell adhesion and durability, particularly water resistance, are readily be achieved. When the thickness of the surface layer 5 is at most the upper limit value of the above range, the surface layer will be excellent in strength. The thickness of the surface layer 5 is obtained by measurement by an X-ray reflectance measuring apparatus represented by ATX-G manufactured by Rigaku Corporation.

As a method of forming the surface layer 5 using the composition (Y) on a predetermined region on the upper surface of the bottom 3, dry coating or wet coating may be mentioned, and dry coating is preferred.

As dry coating, vacuum deposition, CVD, sputtering or the like may be mentioned. With a view to suppressing decomposition of the compound (X) and simplicity of the apparatus, vacuum deposition method is suitably utilized. The vacuum deposition method may be classified into resistance heating method, electron beam heating method, high frequency induction heating method, reactive deposition method, molecular beam epitaxy method, hot wall deposition method, ion plating method, cluster ion beam method, etc., and any method is applicable. With a view to suppressing decomposition of the compound (X) and in view of simplicity of the apparatus, resistance heating method is suitably employed. The vacuum deposition apparatus is not particularly limited, and a known apparatus may be used.

The film deposition conditions when the vacuum deposition method is employed vary depending upon the type of the vacuum deposition method employed, and in the case of the resistance heating method, the degree of vacuum before deposition is preferably at most 1×10⁻² Pa, particularly preferably at most 1×10⁻³ Pa. The heating temperature of the deposition source is not particularly limited so long as the deposition source (the composition (Y) for dry coating) has a sufficient vapor pressure. It is specifically preferably from 30 to 400°C, particularly preferably from 50 to 300°C.

In a case where the heating temperature is at least the lower limit value of the above range, the film deposition rate will be favorable. When it is at most the upper limit value of the above range, the surface layer 5 can be formed on a predetermined region on the upper surface 3a of the bottom 3 without decomposition of the compound (X). The temperature of the bottom 3 at the time of vacuum deposition is preferably within a range of from room temperature (20 to 25°C) to 200°C. When the temperature of the bottom 3 is at least room temperature, the film deposition rate will be favorable. When the temperature of the bottom 3 is at most 200°C, the surface layer can be formed on the substrate without condensation reaction, and the surface layer 5 will have a covalent bond to the substrate quickly after the film deposition. The upper limit value of the temperature of the bottom 3 is more preferably 100°C.

When the dry coating method is conducted, deposition of the composition (Y) on the predetermined region on the upper surface 3a of the bottom 3 is carried out so that the amount of the compound (X) deposited will be from 0.5 to 10 mg/m² so that the obtainable surface layer 5 has the above preferred thickness. The amount of the compound (X) deposited is more preferably from 0.5 to 5 mg/m², particularly preferably from 1.0 to 5.0 mg/m².

When the dry coating method is conducted, the reaction of the compound (X) proceeds substantially simultaneously with the film deposition by adjusting the temperature of the bottom 3 as above. On that occasion, some of silanol groups formed by the hydrolysis reaction from the alkoxysilyl group of the compound (X) undergo condensation reaction to form an intermolecular bonding. The silanol groups formed from the compound (X) undergo condensation reaction with the glass material-OH group which the upper surface 3a of the bottom 3 has, whereby the bottom 3 and the surface layer 5 are bonded by a covalent bond.

As a method of forming the surface layer by wet coating, a method comprising coating a predetermined surface of the bottom 3 with the above-described composition (Y) containing the liquid medium to obtain a coating film (hereinafter sometimes referred to as "coating step") and curing the coating film to obtain a surface layer (hereinafter sometimes referred to as "curing step") may be mentioned.

In the coating step, the method of coating the surface of the bottom 3 with the composition (Y), for example, dip coating method, spin coating method, wipe coating method, spray coating method, squeegee coating method, die coating method, inkjet method, flow coating method, roll coating method, casting method, Langmuir-Blodgett method or gravure coating method may, for example, be mentioned.

In the curing step, as the method of curing the coating film, heating is preferred. The heating temperature depends on the type of the compound (X) and is preferably from 50 to 200°C, more preferably from 80 to 150°C. In the curing step, usually, the liquid medium is removed at the same time. Accordingly, the heating temperature is preferably at least the boiling point of the liquid medium.

When the surface layer 5 is formed by wet coating, a step other than the coating step and the drying step may be conducted as the case requires. For example, in a case where the composition (Y) contains no water, humidification treatment or the like may be conducted simultaneously with or before or after the curing step.

Further, after the surface layer 5 is formed, the compound in excess in the surface layer 5 may be removed as the case requires. As a specific method, for example, a method of rinsing the surface layer 5 with a solvent, for example, the compound used as the liquid medium in the composition (Y), or a method of wiping the surface layer 5 with cloth impregnated with a solvent, for example, the compound used as the liquid medium in the composition (Y) may be mentioned.

The cell culture container of the present invention was described with reference to the cell culture container 10 shown in Figs. 1 and 2 as an example. Further, the surface layer 5 was described with reference to the surface layer 5 formed particularly by using the composition (Y) as an example. Of the cell culture container 10, within the intension and the scope of the present invention, and as the case requires, the constitution may properly be changed. For example, the cell culture container of the present invention may be a cell culture container having a plurality of units, each unit having a constitution having one opening 1 formed by the bottom 3 and the side walls 2 and having a plurality of recesses 4 having the surface layer 5 formed thereon, in the region S on the upper surface 3a of the bottom 3 which the opening 1 faces, as shown in Figs. 1 and 2.

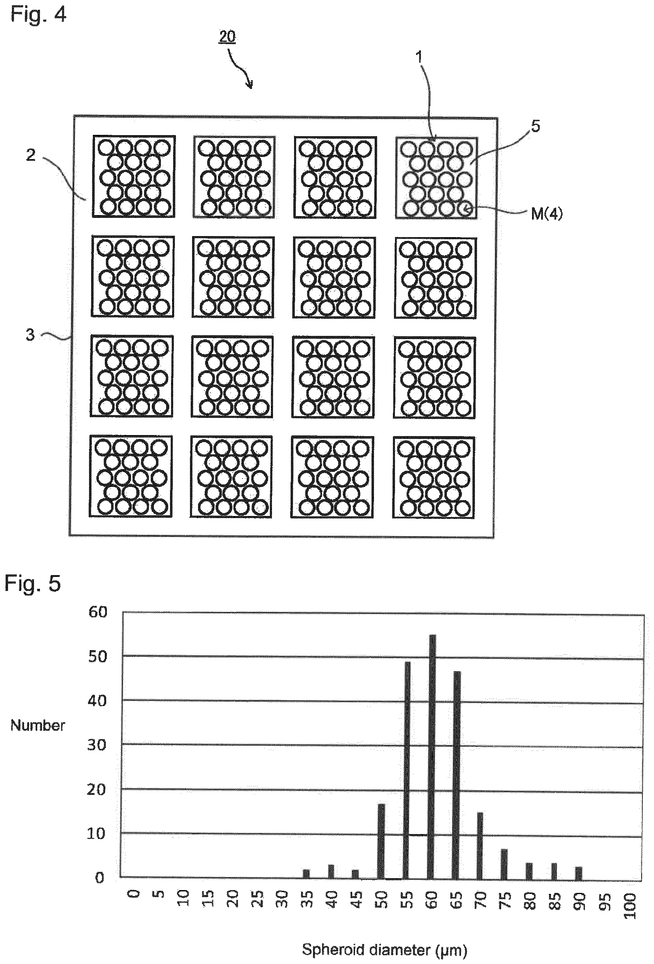

Fig. 4 is a plan view schematically illustrating an example of the cell culture container having a plurality of the above units. The cell culture container 20 shown in Fig. 4 is rectangular in a plan view, and has a constitution having four such units disposed lengthwise at substantially constant intervals as one column, and four such columns disposed crosswise at substantially constant intervals. The respective constituents such as the opening 1, the side walls 2, the bottom 3, the recesses 4, the surface layer and the microspaces M in one unit are as defined for the cell culture container 10. In the cell culture container 20, the side walls 2 are shared by adjacent units. Further, in Fig. 4, the reference symbol M(4) is, in the same manner as M(4) in Fig. 1, a reference symbol represented by the microspace M and the recess 4 in combination.

The size and the shape of the cell culture container 20 in a plan view may be properly adjusted depending upon the application. From the viewpoint of handling efficiency, the shape is preferably rectangular, and the lengthwise and crosswise sizes are preferably each independently within a range of from 75 to 150 mm. The number of the units of the cell culture container 20 is properly adjusted depending upon the size of the cell culture container 20 and the size of the units. The number of the units in the cell culture container 20 is usually from about 6 to about 1,536, preferably from 6 to 384.

In the cell culture container 20, the bottom may, for example, be a glass plate in a substantially planar shape having a periphery of the same shape and size as the periphery of the cell culture container 20, and have a predetermined number of the recesses 4 in the region S corresponding to the respective units on the upper surface 3a. Further, the side walls 2 may be side walls 2 integrally formed in a grid so that a plurality of openings 1 having a predetermined size are formed in a predetermined arrangement on the upper surface 3a of the bottom 3.

The cell culture container of the present invention is subjected to sterilization treatment such as EOG sterilization (sterilization with ethylene oxide gas at 60°C) or autoclave sterilization (sterilization in saturated water vapor at 121°C for 20 minutes) and then used for cell culture. The cell culture container of the present invention is suitable for preparation of spheroids.

For example, in preparation of spheroids using the cell culture container 10, a plurality of cells to be cultured (cell suspension) were charged into the cell culture container 10, and the cell culture container 10 main body is shaken so as to uniformly disperse the plurality of cells in a plurality of microspaces M. Then, cell culture or incubation is conducted in an incubator kept, for example, at 37°C in a saturated water vapor in a 5% carbon dioxide gas atmosphere for several hours to several days.

Since the inner surface of the microspaces M is formed by the surface of the surface layer 5, the cells in the microspaces M are attached to one another without being attached to the inner surface thereby to form spheroids. On that occasion, the cells are three-dimensionally aggregated in accordance with the shape and the size of the microspaces M. Thus, in the cell culture container of the present invention, spheroids having a uniform size can be obtained highly efficiently.

Further, the bottom 3 comprises a glass material, whereby after preparation of the spheroids, the cell culture container 10 is capable of high precision observation as it is by microscopic observation, particularly fluorescence microscopic observation.

Considering application to drug discovery screening of preparing cardiac muscle cell or cancer cell spheroids and examining medicinal effects and toxicity, the proportion of the average diameter ±5% of spheroids, which is an index representing the uniformity of the size, is preferably at least 20%, more preferably at least 30%, particularly preferably at least 50%.

### EXAMPLES

Now, the present invention will be described in detail with reference to Examples. However, it should be understood that the present invention is by no means restricted to the following description. "%" means "mass%" unless otherwise specified. Ex. 1 to 9 are examples for preparation of a bottom with a surface layer (provided that only bottom in Ex. 9), Ex. 1 to 7 are Examples of the present invention, and Ex. 8 and 9 are Comparative Examples. Ex. 11 to 19 are Examples of the present invention regarding the cell culture container.

### (Formation of recesses on upper surface of glass substrate)

In the following method, two types of bottoms having a constitution substantially the same as the bottom 3 of the cell culture container 20 shown in Fig. 4 except that the following predetermined number of the regions S each having a constitution of the recesses substantially the same as that in Figs. 3A and 3B but differing in the number, size, etc. of the recesses as follows, were disposed.

### <Constitution 1 of bottom; bottom 3X>

The bottom 3X has a constitution such that 384 regions S having the following recess constitution 1 are disposed on one principal plane of a glass substrate of 108 mm x 75 mm x 0.6 mm in thickness. The respective regions S are disposed so that side walls are formed in a grid on a flat region Sf among the regions S.

In the recess constitution 1, the region S is 3.0 mm × 3.0 mm, the shape of each recess 4 is semisphere, the number of the recesses 4 in the region S is 156, the depth H of the recess 4 is 100 µm, the diameter Dh of the recess opening surface 4a is 200 µm, the distance Dx between centers of the recess opening surfaces 4a of adjacent recesses 4 is 240 µm, and Dx/Dh is 1.2.

### <Constitution 2 of bottom; bottom 3Y>

The bottom 3Y has a constitution such that 96 regions S having the following recess constitution 2 are disposed on one principal plane of a glass substrate of 108 mm × 75 mm × 0.6 mm in thickness. The respective regions S are disposed so that side walls are provided in a grid on a flat region Sf among the regions S.

The recess constitution 2 is such that the region S is 12.0 mm x12.0 mm, the shape of each recess 4 is semisphere, the number of the recesses 4 in the region S is 250, the depth H of the recess 4 is 250 µm, the diameter Dh of the recess opening surface 4a is 500 µm, the distance Dx between centers of the recess opening surfaces 4a of adjacent recesses 4 is 500 µm, and Dx/Dh is 2.0.

### <Formation of recesses on glass substrate>

Using a glass substrate (Dragontrail (registered trademark), manufactured by AGC Inc., 108 mm in length, 75 mm in width, 0.6 mm in thickness) having a Cr layer on both principal planes, on the Cr layer on both principal planes, a photosensitive resin composition (tradename: Glibes N-100, manufactured by TOKYO OHKA KOGYO CO., LTD.) was applied by a spin coater, followed by soft baking and prebaking. Then, one principal plane was irradiated with ultraviolet rays via a Cr mask such that the non-exposed portions corresponded to the recess opening surfaces 4a, and the whole surface of the other principal plane without the mask, to conduct post-exposure baking. Then, the non-exposed portions were dissolved and removed using a developer to remove the regions to be recesses thereby to obtain a glass substrate having a cured film (protective film) of the photosensitive resin composition formed thereon.

The entire glass substrate with a protective film obtained was dipped in an etching liquid of hydrogen fluoride/sulfuric acid/water = 15/15/70 (mass ratio) and shaken in the etching liquid to conduct etching until recesses 4 having a desired size were formed. Then, the glass substrate with a protective film after the etching was dipped in a release agent to remove the cured film of the photosensitive resin composition and the Cr layer thereby to obtain a bottom 3X and a bottom 3Y each having recesses 4 having predetermined size and shape on a predetermined region on one principal plane to be the upper surface 3a.

### (Synthesis, preparation of compound (X))

### <Compound (X1)>

Compounds classified into the compound (X1) and a comparative compound having no biocompatible group were synthesized or prepared as follows.

Compound (X11-1): as compound (X11-1) having the following structure, that is, 2-[methoxy(polyoxyethylene)₉₋₁₂propyl]trimethoxysilane, commercial product SIM6492.72 (tradename, manufactured by GELEST, INC.) was prepared. Compound (X11-1) is a compound of the formula (X11) wherein the terminal hydrogen atoms is substituted by a methyl group, n1 is from 9 to 12, Q¹ is -C₃H₆-, t is 3, and R⁸ is a methyl group.

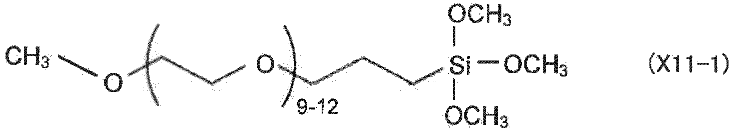

Compound (X11-2): as compound (X11-2) having the same molecular structure as the compound (X11-1) except that the repetition number of the oxyethylene groups is from 6 to 9, that is, 2-[methoxy(polyethyleneoxy)₆₋₉propyl]trimethoxysilane, commercial product SIM6492.7 (tradename, manufactured by GELEST, INC.) was prepared.

Compound (X12-1): compound (X12-1) having the following structure is a compound (X12) wherein n is from 7 to 8, Q¹ is -CONHC₃H₆-, t is 3, and R⁸ is an ethyl group, and was synthesized as follows.

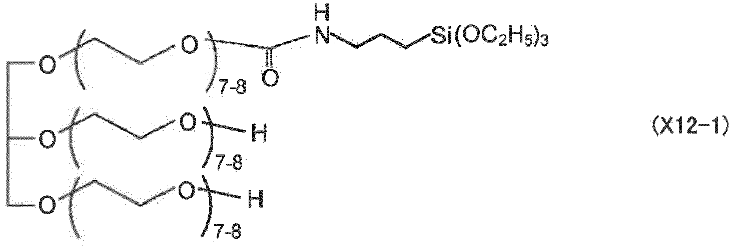

Into a 300 mL eggplant flask, 263 mg (259 mmol) of polyoxyethylene glyceryl ether wherein n1 is from 7 to 8 (in Table 1, represented as "polyoxyethylene polyol A"), 64.1 g (259 mmol) of KBE-9007 (manufactured by Shin-Etsu Silicone, tradename, triethoxysilylpropyl isocyanate) were added. Then, 3.27 g (32.4 mmol) of triethylamine in an amount of 1 mass% of the obtained mixture was added, followed by stirring at 80°C for 16 hours. Then, the obtained reaction mixture was distilled by a rotary evaporator with heating under reduced pressure to remove triethylamine thereby to obtain compound (X12-1) as a colorless transparent liquid. The amount obtained was 327 g, and the yield was 100%.

Compound (Cf1): as compound (Cf1), (3-methoxypropyl)trimethoxysilane (CH₃-O-(CH₂)₃-Si(OCH₃)₃), commercial product, SIM6493.0 (tradename, manufactured by GELEST, INC.)) was prepared.

The type of the polyoxyethylene polyol used to synthesize the compound (X12-1) and the amount (equivalent) added of KBE-9007 to the polyoxyethylene polyol, and of the above respective compounds, Mw, the repetition number (n1) of (CH₂CH₂O) in the group 1(4), the proportion (mol%) of the group 1 being the group 1 in the group 4, the proportion (mass%) of the biocompatible group (the group 1(4)) in the compound, and the proportion (mass%) of the alkoxysilyl group are shown in Table 1.

**[Table 1]**

| Compound (abbreviation) | | | (X11-1) | (X11-2) | (X12-1) | (Cf1) |
|---|---|---|---|---|---|---|
| Reaction conditions | Type of polyoxyethylene polyol | | Commercial product | Commercial product | A | Commercial product |
| | Amount (equivalent) of KBE-9007 to polyoxyethylene polyol | | | | 1 | |
| Properties of compound | Mw | | 590 | 459 | 1260 | 194.3 |
| | n1 | | 9-12 | 6-9 | 7-8 | 0 |
| | Proportion of group 1 being group 1 in group 4 | mol% | 100 | 100 | 67 | 0 |
| | Proportion of biocompatible group | mass% | 69.8 | 61.1 | 77.0 | 0 |
| | Proportion of alkoxysilyl group | mass% | 20.5 | 26.4 | 12.9 | 62.4 |
| Mw after partial hydrolytic condensation | | | - | - | 2730 | 700 |

### <Copolymer (X21)>

As the compound (X2), copolymers (X21-1) to (X21-3) were produced in the following monomer composition (mass ratio) shown in the following Table 2. Further, homopolymer (M) (but not the compound (X)) of a monomer having a biocompatible group was produced. The monomers and their abbreviations are shown below. The monomer composition shown in Table 2, for example, HEMA/KBM-503 = 95/5 in Production Example 2 represents that HEMA and KBM-503 were used in a mass ratio of 95:5. The same applies to the other Production Examples.

### <Monomer abbreviations>

### (1) Monomer (A)

KBM-503: manufactured by Shin-Etsu Silicone, tradename: trimethoxysilylpropyl methacrylate (CH₂=C(CH₃)-COO-(CH₂)₃-Si(OCH₃)₃)
KBM-5103: manufactured by Shin-Etsu Silicone, tradename: trimethoxysilylpropyl acrylate (CH₂=CH-COO-(CH₂)₃-Si(OCH₃)₃)

### (2) Monomer (B1)

AME-400: Blemmer AME-400 (manufactured by NOF CORPORATION, tradename: CH₂=CH-COO-(CH₂CH₂O)₉-CH₃)
HEMA: CH₂=C(CH₃)-COO-CH₂CH₂O-H
HEA: CH₂=CH-COO-CH₂CH₂O-H

### [Production Example 1]

Into a 500 mL three-necked flask, 57.0 g (438 mmol) of HEMA, 3.00 g (12.1 mmol) of KBM-503, 119 g of 1-methoxy-2-propanol, 21 g of diacetone alcohol and 600 mg (2.61 mmol) of dimethyl 2,2'-azobis(2-methyl propionate) were added. The concentration of the monomers in the reaction liquid was 30 mass%, and the initiator concentration was 1 mass%. Then, the obtained mixture was stirred at 75°C in a nitrogen atmosphere for 16 hours and air-cooled to room temperature to obtain a colorless transparent liquid (solution containing 30 mass% of copolymer (X21-1)). The amount obtained was 200 g, and the yield was 100%.

### [Production Examples 2 to 4]

In the same manner as in Production Example 1 except that the monomer composition was changed as identified in Table 2, copolymers (X21-2) and (X21-3) were produced. Further, homopolymer (M) of a monomer having a biocompatible group was produced.

In the compounds (copolymers) obtained in Production Examples 1 to 4, Mw, the repetition number (n2) of (CH₂CH₂O) in the group 1(4), the proportion (mass%) of the biocompatible group (group 1(4)) in the compound and the proportion (mass%) of the alkoxysilyl group are shown in Table 2.

**[Table 2]**

| Production Example | Compound (copolymer) abbreviation | Monomer composition (mass ratio) | Properties of compound | | | | Mw after partial hydrolytic condensation |
|---|---|---|---|---|---|---|---|
| | | | Mw | n2 | Proportion of biocompatible group (mass%) | Proportion of alkoxysilyl group (mass%) | |
| 1 | (X21-1) | HEMA/KBM-503=95/5 | 43200 | 1 | 43.8 | 2.4 | 93600 |
| 2 | (X21-2) | HEA/KBM-5103=95/5 | 38500 | 1 | 49.1 | 2.6 | 71400 |
| 3 | (X21-3) | AME-400/KBM-5103=95/5 | 65200 | 9 | 82.9 | 2.6 | 135000 |
| 4 | (M) | HEMA=100 | 41000 | 1 | 46.1 | 0 | - |

### [Ex. 1]

The above prepared two types of bottoms 3X and 3Y differing in the recess constitution were washed, and on the surface on which the recesses 4 were formed, a surface layer 5 having a film thickness of 2 nm was formed from the compound (X11-1) by vacuum deposition (back pressure: 3.4×10⁻⁴ Pa, substrate temperature: 25°C) to obtain bottom AX with a surface layer and bottom AY with a surface layer respectively from the bottom 3X and the bottom 3Y.

### [Ex. 2]

In the same manner as in Ex. 1 except that the compound (X11-2) was used instead of the compound (X11-1), bottom BX with a surface layer and bottom BY with a surface layer were obtained respectively from the bottom 3X and the bottom 3Y.

### [Ex. 3]

A solution (solid content concentration: 30 mass%) containing the copolymer (X21-1) was added to a mixed solvent of 1-methoxy-2-propanol, diacetone alcohol and a 0.1 mass% aqueous nitric acid solution in a mass ratio of 51:9:40 so that the solid content concentration would be 10 mass%, followed by stirring at 50°C for 16 hours to obtain a liquid composition containing a partially hydrolyzed condensate of the copolymer (X21-1). Mw of the obtained partially hydrolyzed condensate is shown in Table 2. Further, the liquid composition was dissolved in a mixed solvent of methoxypropanol and diacetone alcohol in a mass ratio of 85:15 so that the solid content concentration would be 1.0 mass% to obtain a surface layer-forming composition.

The above prepared two type of bottoms 3X and 3Y differing in the recess constitution were washed, and using the surface layer-forming composition, by dip coating method, a coating film of the surface layer-forming composition was formed on the surface on which the recesses 4 were formed of each of the bottoms 3X and 3Y. Then, the bottoms were dried in a hot air circulating oven at 150°C for one hour to form a surface layer 5 having a film thickness of 1.8 nm thereby to obtain bottom CX with a surface layer and bottom CY with a surface layer respectively from the bottom 3X and the bottom 3Y.

### [Ex. 4, 5 and 6]

In the same manner as in Ex. 3 except that the copolymer (X21-1) was changed to the copolymer (X21-2), the copolymer (X21-3) or the compound (X12-1), bottoms DX, EX and FX with a surface layer and bottoms DY, EY and FY with a surface layer were obtained respectively from the bottoms 3X and 3Y. Mws of partially hydrolyzed condensates of the copolymer (X21-2), the copolymer (X21-3) and the compound (X12-1) are shown in Table 2 or 1.

### [Ex. 7]

The homopolymer (M) was dissolved in a mixed solvent of methoxypropanol and diacetone alcohol in a mass ratio of 85:15 so that the solid content concentration would be 1.0 mass% to obtain a surface layer-forming composition. Using the obtained surface layer-forming composition, in the same manner as in Ex. 3, bottom GX with a surface layer and bottom GY with a surface layer were obtained respectively from the bottom 3X and the bottom 3Y.

### [Ex. 8]

In the same manner as in Ex. 3 except that the copolymer (X21-1) was changed to the compound (Cf1), bottom HX with a surface layer and bottom HY with a surface layer were obtained respectively from the bottom 3X and the bottom 3Y. Mw of a partially hydrolyzed condensate of the compound (Cf1) is shown in Table 1.

### [Ex. 9]

As Ex. 9, the above prepared two types of bottoms 3X and 3Y differing in the recess structure were used as they were.

### [Evaluation]

With respect to the above obtained bottoms AX to HX with a surface layer obtained from the bottom 3X, and the bottom 3X, cell non-adhesiveness and the elution amount were evaluated. The results are shown in Table 3.

### (Cell non-adhesiveness)

Each of the bottoms AX to HX with a surface layer obtained from the bottom 3X, and the bottom 3X, was cut into a sample of 23 mm x 25 mm (containing 20 to 30 regions S with the recess constitution 1) and put in a 50 cc glass vial, and 10 cc of IPA was added, followed by ultrasonic cleaning for 10 minutes. IPA was aspirated, and 10 cc of ethanol was put, followed by ultrasonic cleaning for 10 minutes in the same manner, and each sample was dried to prepare an evaluation substrate.

The obtained 23 mm x 25 mm cleaned evaluation substrate was placed on a polystyrene petri dish having a diameter of 35 mm (1000-035, manufactured by AGC TECHNO GLASS CO., LTD.), followed by UV sterilization in a clean bench for 16 hours.

A cell suspension was prepared using MEM having 10% FBS added as a medium so that TIG-3 cells having a cell survival rate of at least 97% at the time of inoculation confirmed would be 130,000 cells in 3 mL. 3 mL of the cell suspension was dispended into the petri dish on which the evaluation substrate was placed to seed the cells, which were cultured in an incubator at 37°C for 24 hours. Then, microscopic observation (10 magnifications) was conducted with respect to three observation regions of 1.8 mm × 1.3 mm each, and adhesion was evaluated based on the following standards by whether the cells elongated or not. A state where the cells elongated elliptically or roundly on the evaluation substrate is defined as cell elongation.

○: No cells attached in all the observation regions.
Δ: Cells partly attached in at least one observation region.
× : Cells attached substantially entirely in all the observation regions.

### (Measurement of elution amount)

Each of the bottoms AX to HX with a surface layer obtained from the bottom 3X, and the bottom 3X, was cut into a specimen of 108 mm × 25 mm (area: 27 cm²), whih was put in a 100 mL glass vial together with 30 mL of distilled water and left at rest at 40°C for 7 days so that TOC was eluted. The TOC concentration [mg/L] in the obtained eluate was measured by a TOC analyzer TNC-6000 (manufactured by TORAY ENGINEERING Co., Ltd.), which was divided by the area (27 cm²) of the specimen to calculate the TOC elution amount [mg/L] per unit area 1 cm² of the surface layer. Since the TOC elution amount from the bottom 3X is 0 mg/L, the obtained TOC elution amount means the TOC elution amount from the surface layer.

**[Table 3]**

| | Surface layer material abbreviation | Bottom with surface layer | ' Cell non-adhesiveness (130,000 cells) | TOC elution amount [mg/L] |
|---|---|---|---|---|
| Ex. 1 | (X11-1) | AX | ○ | 0 |
| Ex. 2 | (X11-2) | BX | ○ | 0 |
| Ex. 3 | (X21-1) | CX | ○ | 8 |
| Ex. 4 | (X21-2) | DX | ○ | 0 |
| Ex. 5 | (X21-3) | EX | Δ | 5 |
| Ex. 6 | (X12-1) | FX | ○ | 4 |
| Ex. 7 | (M) | GX | ○ | 12 |
| Ex. 8 | (Cf1) | HX | × | 0 |
| Ex. 9 | Nil | Bottom 3X | × | 0 |

### [Ex. 11 to 14]

Each of the above obtained bottoms AX to DX with a surface layer having 384 regions S with the recess constitution 1 was cleaned by the same cleaning method as for evaluation of cell non-adhesiveness. Side walls 2X (manufactured by AGC Inc., material: polystyrene) having a height of 10 mm, formed into a grid so as to correspond to the flat region Sf of each of the bottoms AX to DX with a surface layer, having an outer peripheral size of 108×75 mm and having 384 compartments (corresponding to the regions S) compartmentalized by the grid, were bonded by a double-sided adhesive tape to the surface layer of the flat region Sf among the regions S on each of the cleaned bottoms AX to DX with a surface layer, to prepare 384 well microplate type culture containers (the size of the bottom: 108×75 mm, depth: 10 mm) 11 to 14 in Ex. 11 to 14, having substantially the same constitution as the cell culture container 20 shown in Fig. 4 in a plan view and having 384 units.

### [Ex. 15 to 19]

In the same manner as above, each of the bottoms AY and CY to FY with a surface layer having 96 regions S with the recess constitution 2, and side walls 2Y (manufactured by AGC Inc., material: polystyrene) having a height of 10 mm, formed into a grid so as to correspond to the flat region Sf of each of the bottoms AY and CY to FY with a surface layer, having an outer peripheral size of 108×75 mm and having 96 compartments (corresponding to the regions S) compartmentalized by the grid, were bonded to prepare 96 well microplate type culture containers (size of the bottom: 108×75 mm, depth: 10 mm) 15 to 19 in Ex. 15 to 19.

### [Evaluation]

### (Spheroid preparation efficiency)

Into each of the microplate type culture containers 11 to 20, 50 µL of a cell suspension prepared to contain 200,000 cells per 1 mL was dispensed to seed the cells, which were cultured in an incubator at 37°C for 24 hours. The cells in the microplate type culture container were stained with Calcein-AM, and spheroids were observed by a fluorescence microscope. Fluorescence microscopic (5 magnifications) observation images were analyzed by Image-J (manufactured by National Institute of Health (NIH), Ver. 1.47) to quantitatively analyze the sizes of the spheroids in an observation region (2.34 mm²) to calculate the average diameter of the spheroids and the proportion (%) of the spheroid average diameter ±5%. The average diameter of the spheroids was calculated by image analysis by Image J, assuming the spheroids being circles. The results are shown in Table 4.

Fig. 5 illustrates a distribution state of the spheroid diameters evaluated with respect to the cell culture container 11 in Ex. 11. In Fig. 5, for example, the number represented by the spheroid diameter of 60 µm is the number of spheroids having diameters larger than 55 µm and at most 60 mm.

**[Table 4]**

| | Surface layer material abbreviation | Bottom with surface layer | Cell culture container | Proportion (%) of spheroid average diameter ±5% |
|---|---|---|---|---|
| Ex. 11 | (X11-1) | AX | 11 | 55.6 |
| Ex. 12 | (X11-2) | BX | 12 | 55.6 |
| Ex. 13 | (X21-1) | CX | 13 | 19.7 |
| Ex. 14 | (X21-2) | DX | 14 | 29.5 |
| Ex. 15 | (X11-1) | AY | 15 | 39.7 |
| Ex. 16 | (X21-1) | CY | 16 | 23.9 |
| Ex. 17 | (X21-2) | DY | 17 | 35.8 |
| Ex. 18 | (X21-3) | EY | 18 | 10.5 |
| Ex. 19 | (X12-1) | FY | 19 | 15.5 |

As evident from Table 4, using the cell culture containers in Ex. 11 to 19 which are Examples of the present invention, spheroids with a uniform size can be efficiently produced. Further, with the cell culture containers in Ex. 11 to 19, as mentioned above, fluorescence microscopic observation is easily conducted.

### INDUSTRIAL APPLICABILITY

According to the cell culture container of the present invention, spheroids having a uniform size can be efficiently prepared, and microscopic observation, particularly fluorescence microscopic observation can be conducted simply. Accordingly, the present invention is useful for drug discovery screening to examine medicinal effects and toxicity.

The entire disclosure of Japanese Patent Application No. 2018-016737 filed on February 1, 2018 including specification, claims, drawings and summary is incorporated herein by reference in its entirety.

### REFERENCE SYMBOLS

10, 20: Cell culture container, 1: opening, 2: side wall, 3: bottom, 4: recess, 5: surface layer

## Claims

1. A cell culture container comprising:
side walls forming an opening,
a bottom comprising a light-transmitting glass material, closing the lower end of the opening and having a plurality of recesses in a region which the opening faces on its upper surface, and
a surface layer inhibiting cell adhesion formed on the inner surface of the recesses.

2. The cell culture container according to Claim 1, wherein the proportion of the total area of the recesses in a plan view to the whole area of the region which the opening faces on the upper surface of the bottom in a plan view, is at least 40%.

3. The cell culture container according to Claim 1 or 2, which has a plurality of structures each having the opening and the plurality of recesses having the surface layer formed thereon, in a region which the opening faces on the upper surface of the bottom.

4. The cell culture container according to any one of Claims 1 to 3, wherein a value obtained by dividing the fluorescence intensity at 584 nm when the glass material is excited by light at 532 nm by the fluorescence intensity at 584 nm when quartz glass is excited by light at 532 nm, measured by a 100 magnification objective lens by means of microscopic Raman spectroscopy, is at most 10.

5. The cell culture container according to any one of Claims 1 to 4, wherein the surface layer has a covalent bond with the bottom.

6. The cell culture container according to any one of Claims 1 to 5, wherein the amount of the total organic carbon (TOC) eluted from the surface layer into water per unit area 1 cm² of the surface layer, when the surface layer is dipped in water at 40°C for 7 days, is at most 10 mg/L.

7. The cell culture container according to any one of Claims 1 to 6, which has a silicon oxide layer between the surface layer and the bottom.

8. The cell culture container according to any one of Claims 1 to 7, wherein the surface layer has a biocompatible group.

9. The cell culture container according to Claim 8, wherein the biocompatible group comprises at least one type selected from the group consisting of a group represented by the following formula 1, a group represented by the following formula 2 and a group represented by the following formula 3: 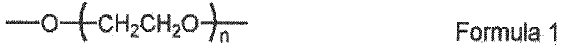 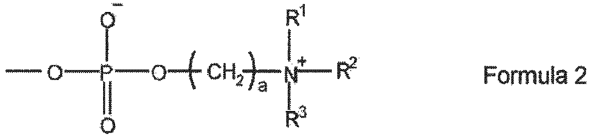 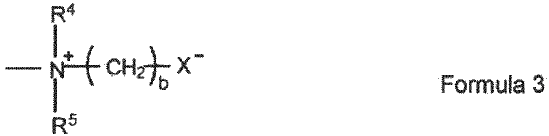 in the formula 1, n is an integer of from 1 to 300, and from 50 to 100 mol% of the group represented by the formula 1 is the group represented by the formula 1 in a group represented by the following formula 4, n in the formula 4 is an integer of from 1 to 300, and R⁶ is a hydrogen atom or a C₁₋₅ alkyl group,
in the formula 2, R¹ to R³ are each independently a C₁₋₅ alkyl group, and a is an integer of from 1 to 5, and
in the formula 3, R⁴ and R⁵ are each independently a C₁₋₅ alkyl group, X⁻ is a group represented by the following formula 3-1 or a group represented by the following formula 3-2, and b is an integer of from 1 to 5:

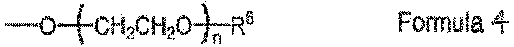

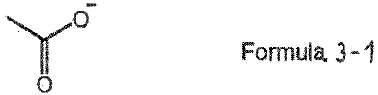

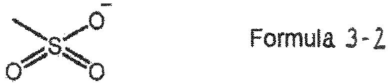

10. A drug discovery screening method, using the cell culture container as defined in any one of Claims 1 to 9.
